(19) Europäisches Patentamt
European Patent Office
Office européen des brevets

(11) Numéro de publication : **0 329 563 B1**

# (12) FASCICULE DE BREVET EUROPEEN

(45) Date de publication du fascicule du brevet : **21.04.93 Bulletin 93/16**

(21) Numéro de dépôt : **89400450.6**

(22) Date de dépôt : **17.02.89**

(51) Int. Cl.$^5$ : **C07D 295/135,** C07D 295/155, A61K 31/40, C07D 333/60, A61K 31/38

## (54) Nouveaux dérivés du benzocyclohexane et du benzocycloheptane ainsi que leurs sels, leurs procédé et intermédiaires de préparation, leur application comme médicaments et les compositions les renfermant.

Le dossier contient des informations techniques présentées postérieurement au dépôt de la demande et ne figurant pas dans le présent fascicule.

(30) Priorité : **18.02.88 FR 8801928**

(43) Date de publication de la demande : **23.08.89 Bulletin 89/34**

(45) Mention de la délivrance du brevet : **21.04.93 Bulletin 93/16**

(84) Etats contractants désignés : **CH DE FR GB IT LI NL**

(56) Documents cités :
**EP-A- 0 129 991**
**EP-A- 0 147 085**
**EP-A- 0 260 555**
**FR-A- 2 370 723**
**US-A- 3 836 534**

(56) Documents cités :
**CHEMICAL AND PHARMACEUTICAL BULLETIN, vol. 26, no. 2, 1978, pages 394-404, Tokyo, JP; H. SUGIHARA et al.: "Syntheses of 1,2-N-Alkylimino-1,2,3,4-tetrahydronaphthalene derivatives and preparation of ring closed analog of salbutamol as a new beta-adrenoceptor agent"**
**BULLETIN DE LA SOCIETE CHIMIQUE DE FRANCE, no. 5, 1971, pages 1649-1657, Paris, FR; J.-J. BARIEUX et al.: "L'hydroboration des énamines et ses applications. 1. - Déplacement du carbonyle de quelques cyclanones symétriquement substituées"**

(73) Titulaire : **ROUSSEL-UCLAF**
**35, Boulevard des Invalides**
**F-75007 Paris (FR)**

(72) Inventeur : **Clemence, François**
**2, Rue Turgot**
**F-75009 Paris (FR)**
Inventeur : **Fortin, Michel**
**12, Passage Cottin**
**F-75018 Paris (FR)**
Inventeur : **Frechet, Daniel**
**45, Rue Lecourbe**
**F-75015 Paris (FR)**
Inventeur : **Moura, Anne-Marie**
**15-29, Rue Guilleminot**
**F-75014 Paris (FR)**

(74) Mandataire : **Bourgouin, André et al**
**Département des Brevets ROUSSEL UCLAF**
**111, route de Noisy B.P. no 9**
**F-93230 Romainville (FR)**

EP 0 329 563 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne de nouveaux dérivés du benzocyclohexane et du benzocycloheptane ainsi que leurs sels, leur procédé de préparation et les nouveaux intermédiaires ainsi obtenus, leur application comme médicaments et les compositions les renfermant.

La demande EP 0260555 décrit des dérivés cycliques formés d'un radical phényl fusionné avec un radical cycloalkyle. Cependant cette demande n'était pas publiée à la date prioritaire de la présente demande.

La demande EP 0129991 décrit des dérivés 2-aminocycloalkényle carboxamides.

La demande FR 2370723 décrit des arylacylamides N-(2-aminocycloaliphatiques).

Tous les produits décrits dans ces références sont différents des produits de la présente demande.

L'invention a pour objet les composés de formule (I) :

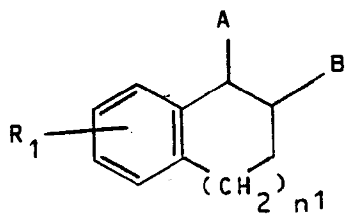

(I)

dans laquelle $R_1$ représente un atome d'hydrogène, un atome d'halogène, un radical alkyle ou alcoxy renfermant de 1 à 5 atomes de carbone, nitro, amino, monoalkylamino ou dialkylamino, $n_1$ représente 1 ou 2, et A et B sont en configuration trans et sont tels que l'un des substituants A ou B est choisi dans le groupe de formule :

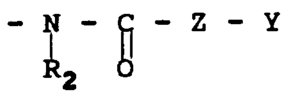

$R_2$ étant un atome d'hydrogène ou un radical alkyle renfermant de 1 à 5 atomes de carbone, Z une chaîne alkylène linéaire - $(CH_2)_{n_2}$-, $n_2$ étant un nombre entier pouvant varier entre O et 5 ou une chaîne alkylène ramifiée renfermant de 2 à 8 atomes de carbone ou un groupement $-CH_2-O-$, Y représente un radical phényle éventuellement substitué par un ou plusieurs radicaux alkyle ou alcoxy renfermant de 1 à 5 atomes de carbone, atomes d'halogène, radicaux trifluorométhyle, nitro, amino, monoalkyle ou dialkylamino ; un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, et l'autre des substituants A ou B est choisi dans le groupe de formule :

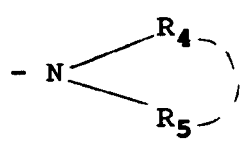

$R_4$ et $R_5$, identiques ou différents, représentant un atome d'hydrogène ou un radical alkyle renfermant de 1 à 5 atomes de carbone ou $R_4$ et $R_5$ forment ensemble avec l'atome d'azote auquel ils sont liés un radical pyrrolidinyle, piperazinyle, piperidinyle, morpholinyle, et à la condition que

ou bien A représente un groupe de formule

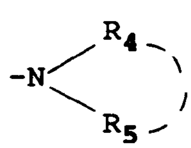

dans lequel $R_4$ et $R_5$ ont la signification précédente et B représente un groupe de formule

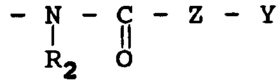

dans lequel $R_2$, Z et Y ont la signification précédente ;
ou bien Z représente $-(CH_2)n_2-$ dans lequel $n_2$ est 0, 2, 3, 4 ou 5, ou une chaîne alkylène ramifiée renfermant de 2 à 8 atomes de carbone ou un groupement $-CH_2-O-$ ;
ou bien Y représente un radical phényle substitué par un ou plusieurs radicaux alkyle renfermant de 1 à 5 atomes de carbone, alkoxy renfermant de 2 à 5 atomes de carbone, amino, monoalkyle ou dialkylamino ou Y représente un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, à l'exception du benzothiophène non substitué ;
ou bien $R_1$ représente un radical nitro ;
ou bien $R_2$ représente un radical alkyle renfermant 4 ou 5 atomes de carbone ;
ou bien $R_1$ représente un atome d'hydrogène, $n_1$ représente 1, A représente le groupement

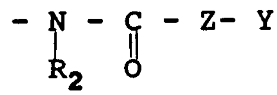

dans lequel $R_2$ est $CH_3$, Z est $CH_2$ et Y est un radical 3,4-diméthoxy-phényle, 4-nitrophényle ou 4-benzothiényle et B représente le radical pyrrolidinyle ;
ou bien $R_1$ représente un atome d'hydrogène, $n_1$ représente 2, A représente le groupement

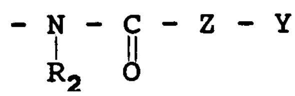

dans lequel $R_2$ est $CH_3$, Z est $CH_2$ et Y est un radical 3,4-diméthoxy-phényle, 3,4-dichlorophényle, 4-trifluorométhylphényle, 4-nitrophényle ou 4-benzothiényle et B représente le radical pyrrolidinyle ; lesdits composés de formule (I) pouvant être dans toutes les formes énantiomères et diastéréoisomères possibles et sous forme de sels d'addition avec les acides.

Lorsque $R_1$ est un radical alkyle et lorsque Y est un radical phényle susbtitué par un ou plusieurs radicaux alkyle, celui-ci est de préférence un radical méthyle ou éthyle, n-propyle ou isopropyle, mais ce substituant peut également représenter un radical n-butyle, isobutyle, n-pentyle.

Lorsque $R_1$ est un radical alkoxy et lorsque Y est un radical phényle substitué par un ou plusieurs radicaux alkoxy, celui-ci est de préférence un radical méthoxy ou éthoxy, mais ce substituant peut aussi représenter un radical propoxy, isopropoxy, butoxy linéaire, secondaire ou tertiaire.

Lorsque $R_1$ est un atome d'halogène et lorsque Y est un radical phényle substitué par un ou plusieurs atomes d'halogène, celui-ci est de préférence un atome de chlore mais il peut aussi représenter un atome de fluor, de brome ou d'iode.

Lorsque les substituants sont des radicaux monoalkyle ou dialkylamino, il s'agit de préférence des radicaux monoalkyle ou dialkylamino dans lesquels les radicaux alkyle sont les radicaux méthyle ou éthyle.

Lorsque Z représente un groupe $-(CH_2)_n$, n est de préférence égal à 0 ou à 1.

Lorsque Z est une chaîne alkylène ramifiée, il s'agit de préférence d'une chaîne substituée par un ou plusieurs radicaux méthyle ou éthyle. Il s'agit par exemple de la chaîne 1,1-éthanediyl ; méthyl-1 éthanediyl-1,2 ; méthyl-1 ou 2 propanediyl-1,2 ; éthyl-1 éthanediyl-1,2.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne plus particulièrement les composés de formule (I) caractérisés en ce que dans le groupe

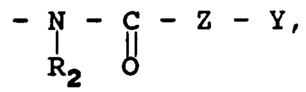

$R_2$ est un radical méthyle ou éthyle, Z un groupe $(CH_2)_{n_2}$ - dans lequel $n_2$ est 0 ou 1,

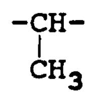

ou $-CH_2-O-$, et en ce que dans le groupe

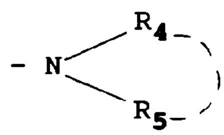

$R_4$ et $R_5$ forment avec l'atome d'azote auquel ils sont liés l'hétérocycle pyrrolidine, ainsi que leurs sels d'addition avec les acides.

Elle a plus particulièrement pour objet les composés de formule (I) caractérisés en ce que dans le groupe

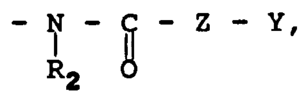

Y représente un radical phényle substitué éventuellement par un ou deux radicaux identiques choisis parmi les radicaux méthyle ou éthyle, méthoxy ou éthoxy, les atomes de chlore ou de brome, les radicaux trifluorométhyle ou nitro, ou Y représente un radical naphtyle ou benzo[b] thiophène, ainsi que leurs sels d'addition avec les acides.

Parmi les composés de formule (I), l'invention a aussi en particulier pour objet ceux pour lesquels ou bien $R_1$ représente un atome d'hydrogène, $n_1$ représente 1, A représente le groupement

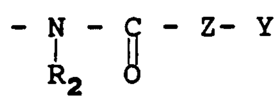

dans lequel $R_2$ est $CH_3$, Z est $CH_2$ et Y est un radical 3,4-diméthoxy-phényle, 4-nitrophényle ou 4-benzothiényle et B représente le radical pyrrolidinyle ;

ou bien $R_1$ représente un atome d'hydrogène, $n_1$ représente 2, A représente le groupement

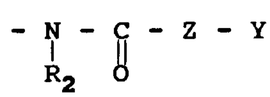

dans lequel $R_2$ est $CH_3$, Z est $CH_2$ et Y est un radical 3,4-diméthoxy-phényle, 3,4-dichlorophényle, 4-trifluorométhylphényle, 4-nitrophényle ou 4-benzothiényle et B représente le radical pyrrolidinyle ; ainsi que leurs sels d'addition avec les acides.

Parmi les composés de formule (I), l'invention a plus particulièrement pour objet les composés de formule (I) :

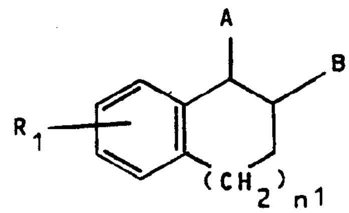

(I)

dans laquelle $R_1$ représente un atome d'hydrogène, un atome d'halogène, un radical alkyle ou alcoxy renfermant de 1 à 5 atomes de carbone, nitro, amino, monoalkylamino ou dialkylamino, $n_1$ représente 1 ou 2, et A et B sont en configuration trans et sont tels que l'un des substituants A ou B représente un radical de formule :

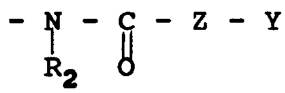

$R_2$ représente un atome d'hydrogène ou un radical alkyle renfermant de 1 à 5 atomes de carbone, Z représente une chaîne alkylène linéaire $-(CH_2)_{n_2}-$, $n_2$ étant un nombre entier pouvant varier entre O et 5 ou une chaîne alkylène ramifiée renfermant de 2 à 8 atomes de carbone ou un groupement $-CH_2-O-$, Y représente un radical phényle éventuellement substitué par un ou plusieurs radicaux alkyle ou alcoxy renfermant de 1 à 5 atomes de carbone, atomes d'halogène, radicaux trifluorométhyle, nitro, amino, monoalkyle ou dialkylamino ; un radical naphtyle, un radical indényle, un radical thiazolyle, pyridinyle, oxazolyle, isoxazolyle, imidazolyle ou thiényle ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents choisis parmi les radicaux méthyle ou éthyle, méthoxy ou éthoxy, un atome de chlore, brome ou fluor, trifluorométhyle, nitro ou phényle, et l'autre des substituants A ou B représente un radical de formule :

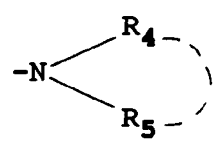

$R_4$ et $R_5$, identiques ou différents, représentant un atome d'hydrogène ou un radical alkyle renfermant de 1 à 5 atomes de carbone ou $R_4$ et $R_5$ forment ensemble avec l'atome d'azote auquel ils sont liés un radical pyrrolidinyle, piperazinyle, piperidinyle, morpholinyle, et à la condition que
ou bien A représente un groupe de formule

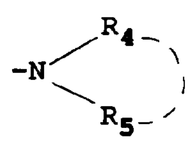

dans lequel $R_4$ et $R_5$ ont la signification précédente et B représente un groupe de formule

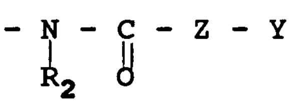

dans lequel $R_2$, Z et Y ont la signification précédente ;
ou bien Z représente $-(CH_2)n_2-$ dans lequel $n_2$ est 0, 2, 3, 4 ou 5, ou une chaîne alkylène ramifiée renfermant de 2 à 8 atomes de carbone ou un groupement $-CH_2-O-$ ;
ou bien Y représente un radical phényle substitué par un ou plusieurs radicaux alkyle renfermant de 1 à 5 atomes de carbone, alkoxy renfermant de 2 à 5 atomes de carbone, amino, monoalkyle ou dialkylamino ou Y représente un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, à l'exception du benzothiophène non substitué ;
ou bien $R_1$ représente un radical nitro ;
ou bien $R_2$ représente un radical alkyle renfermant 4 ou 5 atomes de carbone ;
lesdits composés de formule (I) pouvant être dans toutes les formes énantiomères et diastéréoisomères possibles et sous forme de sels d'addition avec les acides.

Parmi les composés de formule (I), l'invention a tout particulièrement pour objet ceux dont les noms suivent :

- Le trans (±) 4-nitro N-méthyl N-[2-(1-pyrrolidinyl) 1,2,3,4-tétrahydro 1-naphtyl] benzèneacétamide ;
- Le trans (±) N-méthyl N-[2-(1-pyrrolidinyl) 1,2,3,4-tétrahydro 1-naphtyl] 1-naphtalèneacétamide ;
- Le trans (±) N-méthyl N-[2-(1-pyrrolidinyl) 1,2,3,4-tétrahydro 1-naphtyl] 4-benzo[b]thiophèneacétamide ;

ainsi que leurs sels d'addition avec les acides.

L'invention a aussi pour objet un procédé de préparation des produits de formule (I) telle que définie ci-dessus, caractérisé en ce que l'on condense le produit de formule (II) :

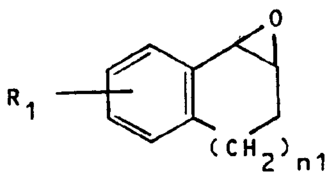

(II)

$R_1$ et $n_1$ ayant les significations données précédemment,

- soit avec une amine de formule (III) :

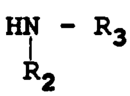

(III)

$R_2$ ayant la signification donnée précédemment et $R_3$ étant un groupement protecteur de la fonction amine pour obtenir un produit de formule (IV) :

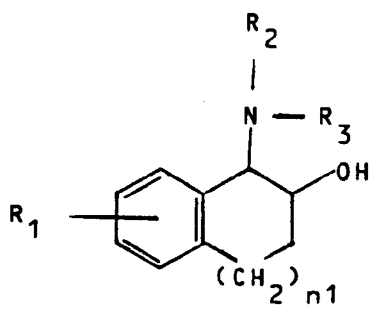

(IV)

dont on active la fonction hydroxyle et que l'on condense avec une amine

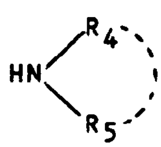

dans laquelle $R_4$ et $R_5$ ont les significations indiquées précédemment, pour obtenir un produit de formule (V) :

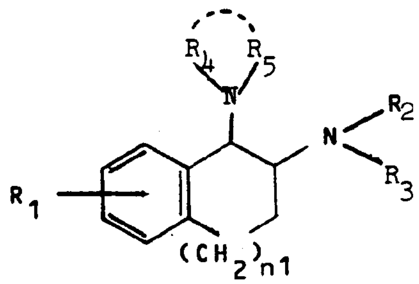

(V)

dont on élimine le groupement protecteur $R_3$ de la fonction amine, pour obtenir un produit de formule (VI) :

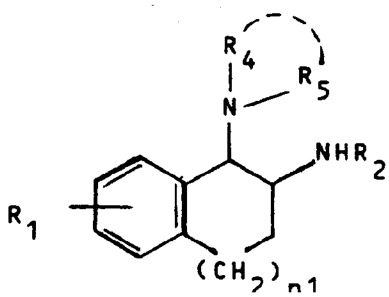

(VI)

que l'on traite avec un acide de formule (VII) ou un dérivé fonctionnel de cet acide :

Y-Z-COOH (VII)

dans laquelle Y et Z ont les significations précédentes, pour obtenir un produit de formule (I) dans laquelle A représente

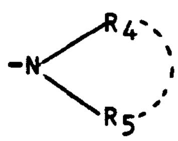 et le B le radical 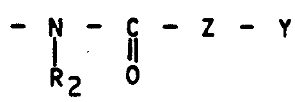 ;

- soit avec une amine

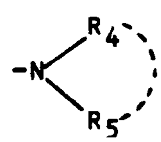

pour obtenir un produit de formule (VIII) :

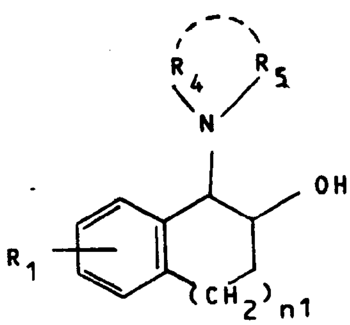

(VIII)

dont on active la fonction hydroxyle et que l'on traite avec une amine de formule (IX) :

$NH_2R_2$ (IX)

dans laquelle $R_2$ a la signification précédente, pour obtenir un produit de formule (X) :

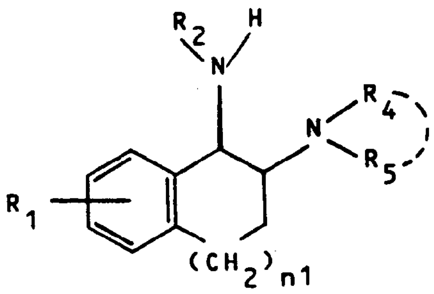

(X)

que l'on condense avec un acide de formule (VII) ou un dérivé fonctionnel de cet acide :

Y-Z-COOH (VII)

dans laquelle Z et Y ont les significations précédentes, pour obtenir un produit de formule (I) dans laquelle A représente le radical

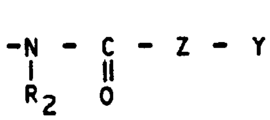 et B le radical 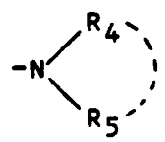 ,

lesdits composés de formule (I) pouvant être dédoublés pour obtenir les formes optiquement actives, et traite, si désiré, avec un acide minéral ou organique, pour obtenir les sels.

Dans les conditions préférentielles du procédé de l'invention :

- Le chlorure de méthanesulfonyle est utilisé pour activer la fonction hydroxyle des produits de formule (IV) et des produits de formule (VIII).
- Dans les produits de formule (V), le groupement de protection $R_3$ est un radical benzyle qui peut être éliminé par hydrogénation catalytique. Le catalyseur que l'on utilise est de préférence le palladium.
- La condensation du composé de formule (VII) avec le composé de formule (VI) ou (X) s'effectue en présence de carbonyl-diimidazole. On peut également utiliser un dérivé fonctionnel de l'acide de formule (VII) tel qu'un chlorure d'acide ou un anhydride mixte.
- Les produits de formule (I) peuvent être dédoublés par les méthodes usuelles.

Les composés de formule (I) tels que définis ci-dessus ainsi que leurs sels d'addition avec les acides présentent d'intéressantes propriétés pharmocologiques. Ils présentent en particulier une forte affinité pour les récepteurs opiacés et notamment pour les récepteurs Kappa et sont doués de propriétés analgésiques centrales.

Ils sont doués également de propriétés diurétiques, de propriétés anti-arythmiques, anti-ischémiques cérébrales et hypotensives.

Ces propriétés justifient leur application en thérapeutique et l'invention a également pour objet à titre de médicaments, les produits tels que définis par la formule (I) ci-dessus ainsi que par leurs sels d'addition avec les acides pharmaceutiquement acceptables.

La présente invention a tout particulièrement pour objet, à titre de médicament :

- le trans (±) 4-nitro N-méthyl N-[2-(1-pyrrolidinyl) 1,2,3, 4-tétrahydro-1-naphtyl] benzène acétamide ;
- le trans (±) N-méthyl N-[2-(1-pyrrolidinyl) 1,2,3,4-tétrahydro-1-naphtyl] 1-naphtalène acétamide ;
- le trans (±) N-méthyl N-[2-(1-pyrrolidinyl) 1,2,3,4-tétrahydro-1-naphtyl] 4-benzo [b] thiophène acétamide ;

ainsi que leurs sels d'addition avec les acides pharmaceutiquement acceptables.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans la traitement des douleurs dentaires, des migraines, du zona, dans le traitement des douleurs intenses, en particulier rebelles aux antalgiques périphériques, par exemple au cours du processus néoplasique, dans le traitement des pancréatiques, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale et

entre 5 et 100 mg par jour, par voie parentérale.

Les médicaments, objet de l'invention, trouvent aussi leur emploi dans le traitement des arythmies.

La dose usuelle, variable selon le dérivé utilisé, le sujet et l'affection en cause, peut être par exemple de 50 mg à 1 g par jour.

Par voie orale, le principe actif peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

Les médicaments, objet de l'invention, peuvent aussi être utilisés dans le traitement des syndromes oedémateux, de l'insuffisance cardiaque, de certaines obésités, des cirrhoses, dans les traitement des oedèmes sévères et réfractaires, en particulier ceux de l'insuffisance cardiaque congestive et dans le traitement au long cours de l'hypertension artérielle.

La dose quotidienne de principe actif est variable. Elle peut être par exemple de 60 à 100 mg par jour par voie orale.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels et les préparations en aérosols ; elles sont préparées seon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifians, les conservateurs.

Par ailleurs, l'invention concerne à titre de produits industriels nouveaux, notamment à titre de produits intermédiaires pour la mise en oeuvre du procédé de l'invention, les produits de formules (V), (VI) et (X) telles que définies ci-dessus et telles que pour les produits de formules (V) et (VI), l'une au moins des conditions suivantes est remplie :

- $R_4$ et $R_5$ ne représentent pas un atome d'hydrogène ;
- $R_1$ représente un radical nitro ;
- $R_2$ représente un radical alkyle ayant 4 ou 5 atomes de carbone et pour les produits de formule (X), l'une au moins des conditions suivantes est remplie :
  - $R_1$ représente un radical nitro ;
  - $R_2$ représente un radical alkyle ayant 4 ou 5 atomes de carbone.

Le produit de formule (II) dans laquelle $n_1 = 1$ et $R_1$ est un atome d'hydrogène, est décrit dans J. Am. Chem. Soc. 101 (19) 5679-87 (1979). Celui pour lequel $n_1 = 2$ et $R_1$ est un atome d'hydrogène est décrit dans J. Org. Chem. Vol. 44 n° 8 1342, (1979).

Lorsque $R_1$ est différent de H, l'époxyde de départ de formule(II)est obtenu non isolé à partir d'un produit bromé sur lequel on fait réagir un hydroxyde de métal alcalin ou d'hydrure de sodium suivant le schéma suivant :

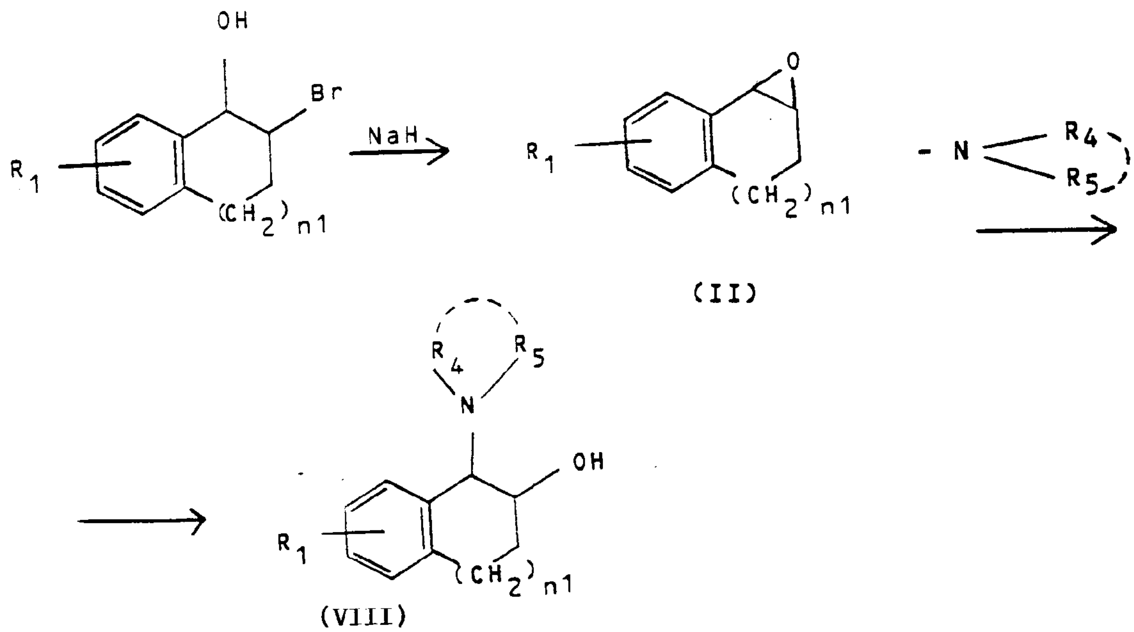


(II)

(VIII)

Les exemples donnés ci-après illustrent l'invention sans toutefois la limiter.

**Exemple 1** : (E) butènedioate de (± trans) 3,4-diméthoxy N-méthyl N-[2-(1-pyrrolidinyl) 1,2,3,4-tétrahydro 1-naphtyl] benzèneacétamide.

Stade A : Trans (±) 1-(1-pyrrolidinyl) 1,2,3,4-tétrahydro 2-naphtol.

On chauffe à 70° C, une solution renfermant 8,8 g de 1a,2,3,7b-tétrahydro naphto[1,2-b]oxirène (J.Am. Chem. Soc. 101(19), 5679-87 (1979)), 18 $cm^3$ d'eau et 18 $cm^3$ de pyrrolidine, pendant 30 minutes, concentre sous pression réduite dans un bain à 50° C, extrait à l'éther, lave à l'eau, sèche, filtre, concentre à 100 $cm^3$, traite au charbon actif, filtre, concentre à sec sous pression réduite dans un bain à 50° C et obtient 10,8 g d'une huile brune.

Stade B : Trans (±) N-méthyl 2-(1-pyrrolidinyl) 1,2,3,4-tétrahydro 1-naphtalènamine.

On refroidit à -20° C, une solution renfermant 10,8 g de produit obtenu au stade A, 100 $cm^3$ de tétrahydrofuranne et 9,7 $cm^3$ de triéthylamine, puis ajoute en 10 minutes 5,5 $cm^3$ de chlorure de méthanesulfonyle. On laisse revenir le précipité obtenu à température ambiante et ajoute ensuite 38 $cm^3$ de méthylamine à 33% dans l'éthanol. On agite 20 heures, élimine le tétrahydrofuranne et l'éthanol sous pression réduite à 50° C, ajoute 100 $cm^3$ d'eau, 10 $cm^3$ de lessive de soude, extraite au chlorure de méthylène, lave à l'eau, sèche, filtre, concentre à sec.
On obtient 11,5 g d'une huile brune.

Stade C : (E) butènedioate de (± trans) 3,4-diméthoxy N-méthyl N-[2-(1-pyrrolidinyl) 1,2,3,4-tétrahydro 1-naphtyl] benzèneacétamide.

On ajoute en une seule fois 3 g de carbonyldiimidazole dans une solution renfermant 4,1 g d'acide 3,4-diméthoxyphényl acétique dans 26 $cm^3$ de tétrahydrofuranne, agite 1 heure à température ambiante, et ajoute rapidement 3 g de produit obtenue au stade B dans 27 $cm^3$ de tétrahydrofuranne. On agite 2 heures à température ambiante, élimine le tétrahydrofuranne sous pression réduite dans un bain à 50° C, reprend avec 70 $cm^3$ d'une solution saturé de carbonate acide de sodium, extrait au chlorure de méthylène, lave à l'eau, sèche, filtre, concentre à sec sous pression réduite à 50° C.
On obtient 6,5 g de produit attendu sous forme de base que l'on dissout dans 60 $cm^3$ d'isopropanol.

On ajoute 1,51 g d'acide fumarique en solution dans 40 $cm^3$ de méthanol, concentre à 70 $cm^3$, amorce la cristallisation, laisse 1 heure à température ambiante, essore, lave à l'isopropanol, sèche sous pression réduite à 90° C. On obtient 4,44 g de produit brut sous forme de sel que l'on purifie par deux recristallisations successives dans l'éthanol.
On obtient 3,13 g de produit attendu sous forme de sel F = 200° C.

| Analyse | C % | H % | N% |
|---|---|---|---|
| Théorie | 66,39 | 6,92 | 5,34 |
| Trouvé | 66,5 | 6,9 | 5,3 |

**Exemple 2** : Trans (±) N-méthyl 4-nitro N-[2-(1-pyrrolidinyl) 1,2,3,4-tétrahydro 1-naphtyl] benzèneacétamide.

On opère comme indiqué au stade C de l'exemple 1 à partir de 770 mg d'acide (p-nitrophényl)acétique et 700 mg de produit obtenu au stade B de l'exemple 1.
On obtient 1,4 g de produit brut que l'on purifie par chromatographie sur silice (éluant : chlorure de méthylène 90-méthanol 10) pour obtient 1,1 g de produit que l'on cristallise dans l'éther éthylique pour obtenir 480 mg de produit.
Deux cristallisations dans l'isopropanol sont effectuées pour obtenir 300 mg de produit attendu F = 135° C.

| Analyse | C % | H % | N % |
|---|---|---|---|
| Théorie | 70,21 | 6,92 | 10,68 |
| Trouvé | 70,3 | 7,0 | 10,6 |

**Exemple 3** : Trans (±) N-méthyl N-[2-(1-pyrrolidinyl) 1,2,3,4-tétrahydro 1-naphtyl] 1-naphtalèneacétamide.

On opère comme indiqué au stade C de l'exemple 1 à partir de 2,3 g d'acide 1-naphtylacétique et de 2 g de produit obtenu au stade B de l'exemple 1.
On obtient 5,3 g de produit brut que l'on purifie par chromatographie sur silice (chlorure de méthylène 9-méthanol 1).
Après cristallisation dans l'éther éthylique, on obtient 1,9 g de produit F = 117° C que l'on recristallise dans l'éther isopropylique pour obtenir 1,1 g de produit attendu F = 125° C.

| Analyse | C % | H % | N % |
|---|---|---|---|
| Théorie | 81,37 | 7,59 | 7,03 |
| Trouvé | 81,7 | 7,6 | 7,0 |

**Exemple 4** : Trans (±) 3,4-dichloro N-méthyl N-[2-(1-pyrrolidinyl) 1,2,3,4-tétrahydro 1-naphtyl] benzamide.

On dissout à température ambiante 478 mg de produit obtenu au stade B de l'exemple 1 dans 15 $cm^3$ d'éther éthylique anhydre, ajoute en 4 fois 870 mg de chlorure de dichloro-3,4 benzoyle, agite 1 heure, essore, lave à l'éther éthylique et obtient 520 mg de produit attendu sous forme de chlorhydrate. Le retour à la base s'effectue en ajoutant 40 $cm^3$ d'eau puis 10 $cm^3$ de soude 2N, en agitant, en extrayant au chlorure de méthylène, en lavant avec de la soude 2N, en séchant et en concentrant à sec sous pression réduite.
On obtient 500 mg de produit que l'on dissout dans 5 $cm^3$ d'acétate d'éthyle. On filtre, concentre, ajoute 2,5 $cm^3$ d'éther isopropylique, amorce la cristallisation et laisse 1 heure à température ambiante. On essore.
On obtient 215 mg de produit attendu F = 138° C.

| Analyse | C % | H % | Cl % | N % |
|---|---|---|---|---|
| Théorie | 65,51 | 6,0 | 17,58 | 6,94 |
| Trouvé | 65,5 | 5,9 | 17,9 | 6,9 |

**Exemples 5 et 6** : Chlorhydrate de [(5alpha, 6béta) (±)] 3,4-dichloro N,alpha-diméthyl N-[6-(1-pyrrolidinyl) 6,7,8,9-tétrahydro 5H-benzocyclohepten-5-yl] benzeneacétamide.

Exemple 5 : Diastéréoisomère A
Exemple 6 : Diastéréoisomère B

Stade A : Trans (±) 5-(1-pyrrolidinyl) 6,7,8,9-tétrahydro-5H-benzocyclohepten-6-ol.

On mélange 6 g de 1a,3,4,8b-tétrahydro 2H-benzo[3,4] cyclohept[1,2,b] oxirène (J. Org. Chem. Vol. 44, N° 8, 1979, 1342) dans 12 $cm^3$ d'eau et ajoute 12 $cm^3$ de pyrrolidine, agite 20 minutes dans un bain à 70° C, concentre à sec sous pression réduite, reprend par 200 $cm^3$ d'éther éthylique, sèche, traite au charbon actif, filtre, concentre à sec sous pression réduite et obtient 8,2 g d'huile orange.

Stade B : Trans (±) N-méthyl 6-(1-pyrrolidinyl) 6,7,8,9-tétrahydro-5H-benzocyclohepten-5-amine.

On dissout 8,2 g de produit obtenu au stade A dans 82 $cm^3$ de tétrahydrofuranne et 7 $cm^3$ de triethylamine. On refroidit par un bain à -20° C et ajoute 3,9 $cm^3$ de chlorure de méthane sulfonyle en solution dans 8 $cm^3$ de tétrahydrofuranne. On laisse revenir à température ambiante, ajoute 35 $cm^3$ de méthylamine à 33 % dans l'éthanol, agite 23 heures, élimine les solvants sous pression réduite, ajoute 150 $cm^3$ d'eau, 10 $cm^3$ de lessive de soude, extrait au chlorure de méthylène, lave à l'eau salée, sèche, filtre, concentre à sec sous pression réduite et obtient 8,7 g d'huile orange.

Stade C : Chlorhydrate de [(5alpha, 6béta) (±)] 3,4-dichloro N, alpha-diméthyl N-[6-(1-pyrrolidinyl) 6,7,8,9-tétrahydro 5H-benzocyclohepten-5-yl] benzeneacétamide.

On dissout 1,6 g d'acide 3,4-dichloro alpha-méthyl benzèneacétique et 1,2 g de 1,1'carbonyldiimidazole dans 16 $cm^3$ de tétrahydrofuranne, agite 30 minutes, et ajoute en une seule fois, 1 g de produit obtenu au stade B en solution dans 16 $cm^3$ de tétrahydrofuranne, agite sous courant d'azote et porte la solution à reflux durant 13 heures 30 minutes.

On élimine le tétrahydrofuranne sous pression réduite, ajoute 50 $cm^3$ de solution saturée de bicarbonate de sodium et 30 $cm^3$ d'eau, extrait au chlorure de méthylène, lave par une solution saturée de chlorure de sodium, sèche, concentre sous pression réduite et obtient 2,45 g de produit brut. On purifie par chromatographie sur silice (éluant : chlorure de méthylène 90-méthanol 10).

On sépare 580 mg de produit présentant un Rf de 0,6 et correspondant au diastéréoisomère A sous forme de base (exemple 5) et 920 mg de produit présentant un Rf de 0,35 correspondant au diastéréoisomère B sous forme de base (exemple 6).

Obtention des chlorhydrates.

On dissout le diastéréoisomère A dans 10 $cm^3$ d'acétate d'éthyle, ajoute 2 $cm^3$ d'une solution acide chlorhydrique-acétate d'éthyle, laisse 48 heures au repos, essore et lave à l'éther éthylique.

On dissout 730 mg de produit ainsi obtenu dans 20 $cm^3$ de chlorure de méthylène, traite au charbon actif, filtre, concentre, ajoute 30 $cm^3$ d'éther éthylique, laisse cristalliser 18 heures, essore, lave à l'éther éthylique et obtient 578 mg de produit attendu.

On dissout dans 100 $cm^3$ d'isopropanol, concentre, laisse au repos 18 heures, essore, lave à l'isopropanol, à l'éther éthylique et sèche sous pression réduite à 80° C.
On obtient 458 mg de produit attendu F = 230° C puis 252° C.

| Analyse | C % | H % | N % | Cl % |
|---|---|---|---|---|
| Calculé | 62,31 | 6,48 | 5,81 | 22,07 |
| Trouvé | 62,2 | 6,5 | 5,6 | 22,0 |

On obtient le chlorhydrate du diastéréoisomère B de façon analogue à celle décrite ci-dessus pour le chlorydrate du diastéréoisomère A.
On obtient 785 mg de produit sous forme de chlorhydrate F = 240° C.

| Analyse | C % | H % | N % | Cl % |
|---|---|---|---|---|
| Calculé | 62,31 | 6,48 | 5,81 | 22,07 |
| Trouvé | 62,4 | 6,5 | 5,8 | 21,9 |

<u>Exemple 7</u> : (E) butènedioate de (±) trans 3,4-diméthoxy N-méthyl N-[6-(1-pyrrolidinyl) 6,7,8,9-tétrahydro 5H-benzocyclohepten-5-yl] benzèneacétamide.

On opère comme au stade C des exemples 5 et 6 à partir de 3,92 g d'acide 3,4-diméthoxy phénylacétique et de 3 g de produit obtenu au stade B des exemples 5 et 6. On obtient 5 g de produit sous forme de base. On y ajoute 75 $cm^3$ d'isopropanol et 1,37 g d'acide fumarique dans 20 $cm^3$ de méthanol. On amorce la cristallisation, laisse une nuit au repos, essore, lave à l'isopropanol et sèche à 100° C sous pression réduite. On obtient 4,3 g de produit attendu sous forme de sel F = 190° C.

| Analyse | C % | H % | N % |
|---|---|---|---|
| Calculé | 66,89 | 7,12 | 5,20 |
| Trouvé | 66,6 | 7,0 | 5,1 |

<u>Exemple 8</u> : (E) 2-butènedioate de trans (±) 3,4-dichloro N-méthyl N-[6-(1-pyrrolidinyl) 6,7,8,9-tétrahydro 5H-benzocyclohepten-5-yl] benzèneacétamide.

On opère comme au stade C des exemples 5 et 6 à partir de 10,5 g d'acide 3,4-dichlorophénylacétique et de 8,7 g de produit obtenu au stade B des exemples 5 et 6. On obtient 16,7 g de produit sous forme de base.
On y ajoute 150 $cm^3$ de méthanol, traite au charbon actif, filtre, ajoute 4,18 g d'acide fumarique dans 100 $cm^3$ de méthanol, amorce la cristallisation, laisse une nuit au repos, essore, lave et sèche sous pression réduite à 90° C. On obtient 15,1 g de produit attendu sous forme de sel F = 170° C puis 210° C.

| Analyse | C % | H % | Cl % | N % |
|---|---|---|---|---|
| Calculé | 61,43 | 5,89 | 12,95 | 5,12 |
| Trouvé | 61,7 | 5,9 | 13,2 | 5,3 |

<u>Exemple 9</u> : Trans (±) N-méthyl N-[6-(1-pyrrolidinyl) 6,7,8,9-tétrahydro 5H-benzocyclohepten-5-yl] 1-naphtalèneacétamide.

On opère comme au stade C des exemples 5 et 6 à partir de 530 mg d'acide 1-naphtylacétique et de 500 mg de produit obtenu au stade B des exemples 5 et 6.
On obtient 1 g d'huile jaune que l'on purifie par chromatographie sur silice (éluant : chlorure de méthylène 90-méthanol 10). On obtient après cristallisation dans l'éther éthylique 613 mg de produit attendu F = 174° C.

| Analyse | C % | H % | N % |
|---|---|---|---|
| Calculé | 81,51 | 7,82 | 6,79 |
| Trouvé | 81,2 | 7,9 | 6,7 |

**Exemple 10** : Chlorhydrate de trans (±) 3,4-dichloro N-méthyl N-[6-(1-pyrrolidinyl) 6,7,8,9-tétrahydro 5H-benzocyclohepten-5-yl] benzamide.

On dissout 244 mg de produit obtenu au stade B des exemples 5 et 6 dans 8 $cm^3$ d'éther éthylique anhydre et ajoute en 4 fois 420 mg de chlorure de dichloro-3,4 benzoyle fraichement préparé, agite une demi-heure, essore, lave à l'éther éthylique, sèche sous pression réduite à 40° C et obtient 408 mg de produit brut sous forme de chlorhydrate.

On les dissout dans 2,5 $cm^3$ de chlorure de méthylène, ajoute 15 $cm^3$ d'éther éthylique, laisse 16 heures au repos, essore et sèche sous pression réduite à 80° C.

On obtient 276 mg de produit que l'on recristallise dans 25 $cm^3$ de méthyléthylcétone et 15 $cm^3$ d'éther éthylique.

On laisse 4 heures au repos, essore, sèche sous pression réduite et obtient 185 mg de produit attendu sous forme de chlorhydrate F = 240° C.

| Analyse | C % | H % | N % | Cl % |
|---|---|---|---|---|
| Calculé | 60,87 | 6,0 | 6,17 | 23,43 |
| Trouvé | 60,8 | 6,1 | 6,2 | 23,5 |

**Exemple 11** : Chlorhydrate de trans (±) N-méthyl N-[6-(1-pyrrolidinyl) 6,7,8,9-tétrahydro 5H-benzocyclohepten-5-yl] 4-(trifluorométhyl)benzèneacétamide.

On opère comme au stade C des exemples 5 et 6 à partir de 580 mg d'acide para-trifluorométhyl phényl acétique et de 492 mg de produit obtenu au stade B des exemples 5 et 6.

On obtient 1,09 g de produit brut que l'on purifie par chromatographie sur silice (chlorure de méthylène 9-méthanol 1) et obtient 750 mg de produit sous forme de base.

On dissout dans 3 $cm^3$ d'acétate d'éthyle, ajoute 2 $cm^3$ d'une solution acide chlorhydrique - acétate d'éthyle (3,5N) et 1,5 $cm^3$ d'éther éthylique, amorce la cristallisation, laisse 48 heures au repos, essore, lave et sèche sous pression réduite à 80° C.

On obtient 659 mg de produit que l'on recristallise 2 fois dans l'éther éthylique.

On obtient 405 mg de produit attendu sous forme de chlorhydrate F = 197° C.

| Analyse | C % | H % | F % | N % | Cl % |
|---|---|---|---|---|---|
| Calculé | 64,3 | 6,47 | 12,2 | 6,0 | 7,59 |
| Trouvé | 64,5 | 6,5 | 12,4 | 5,9 | 8,0-7,9 |

**Exemple 12** : Chlorhydrate de trans (±) N-méthyl N-[6-(1-pyrrolidinyl) 6,7,8,9-tétrahydro 5H-benzocyclohepten-5-yl] 4-nitro benzèneacétamide.

On opère comme au stade C des exemples 5 et 6 à partir de 520 mg d'acide paranitrophénylacétique et de 497 mg de produit obtenu au stade B des exemples 5 et 6.

On obtient 1,08 g d'huile orange que l'on purifie par chromatographie sur silice (éluant : chlorure de méthylène 90-méthanol 10) et obtient 930 mg de produit sous forme de base que l'on transforme en chlorhydrate comme à l'exemple 11.

On obtient 765 mg de produit attendu sous forme de chlorhydrate F = 260° C.

| Analyse | C % | H % | N % | Cl % |
|---|---|---|---|---|
| Calculé | 64,93 | 6,81 | 9,46 | 7,98 |
| Trouvé | 64,9 | 6,9 | 9,5 | 8,0 |

<u>**Exemple 13**</u> : (5alpha, 6béta) (±) N-méthyl N-[6-(1-pyrrolidinyl) 6,7,8,9-tétrahydro 5H-benzocyclohepten-5-yl] benzo[b] thiophène 4-acétamide.

On opère comme au stade C des exemples 5 et 6 à partir de 550 mg d'acide-4-thionaphtène acétique et de 500 mg de produit obtenu au stade B des exemples 5 et 6.
On obtient 1,13 g de produit brut que l'on purifie par chromatographie sur silice (éluant : chlorure de méthylène 90-méthanol 10) pour obtenir 750 mg de produit que l'on cristallise dans de l'éther éthylique pour obtenir 613 mg de produit que l'on recristallise dans le méthanol.
On obtient 400 mg de produit attendu F = 172° C.

| Analyse | C % | H % | N % | S % |
|---|---|---|---|---|
| Calculé | 74,6 | 7,22 | 6,69 | 7,66 |
| Trouvé | 74,6 | 7,3 | 6,7 | 7,7 |

<u>**Exemple 14**</u> : (E) 2-butènedioate de (±)trans 3,4-diméthoxy N-méthyl N-[5-(1-pyrrolidinyl) 6,7,8,9-tétrahydro 5H-benzocyclohepten-6-yl] benzèneacétamide.

<u>Stade A</u> : Trans (±) 5-[méthyl(phénylméthyl)amino] 6,7,8,9-tétrahydro-5H-benzocyclo-hepten-6-ol.

On chauffe à 100° C pendant 1 heure 30 minutes, une solution renfermant 6,8 g de 1a,3,4,8b-tétrahydro 2H-benzo[3,4] cyclohept[1,2,b]oxirène dans 13,6 $cm^3$ d'eau et 13,6 $cm^3$ de benzylméthylamine, concentre presqu'à sec sous pression réduite, extrait le résidu au chlorure de méthylène, lave à l'eau, sèche, filtre, concentre à sec sous pression réduite à 50° C et obtient 13 g d'huile brune que l'on purifie par chromatographie sur silice dans chlorure de méthylène/éther 9/1. On obtient 11 g d'une huile que l'on cristallise dans l'éther isopropylique à - 4° C, essore, lave à l'éther isopropylique, sèche sous pression réduite à 40° C et obtient 4,8 g de produit attendu F = 70° C.

Une chromatographie sur silice (éluant : chlorure de méthylène 9-éther 1) permet de récupérer à partir des liqueurs mères 3,6 g de produit que l'on fait cristalliser également à + 4° C dans l'éther isopropylique. On obtient après essorage 2 g de produit attendu F = 70° C.

<u>Stade B</u> : Trans (±) N-méthyl N-(phénylméthyl) 5-(1-pyrrolidinyl) 6,7,8,9-tétrahydro-5H-benzocyclohepten-6-amine.

On refroidit à - 20° C, une solution contenant 4,2 g de produit préparé au stade A dans 42 $cm^3$ de tétrahydrofuranne anhydre et 2,9 $cm^3$ de triethylamine et ajoute 1,63 $cm^3$ de chlorure de méthane sulfonyle dans 3,25 $cm^3$ de tétrahydrofuranne, laisse revenir à température ambiante et ajoute 7,5 $cm^3$ de pyrrolidine. On agite 24 heures, concentre sous pression réduite à 50° C. On ajoute 90 $cm^3$ d'eau et 10 $cm^3$ de lessive de soude, extrait au chlorure de méthylène, lave à l'eau, sèche, filtre, concentre à sec sous pression réduite et obtient 6,38 g d'huile jaune. On obtient après chromatographie sur silice 5 g de produit (éluant : chlorure de méthylène 95 - méthanol 5) présentant un Rf de 0,3.

<u>Stade C</u> : Trans (±) N-méthyl 5-(1-pyrrolidinyl) 6,7,8,9-tétrahydro-5H-benzocyclohepten-6-amine.

On hydrogène avec 1,25 g de palladium sous 1800 mmbars durant 45 minutes, une solution renfermant 2 g de produit du stade B dans 40 $cm^3$ de méthanol et 2,6 $cm^3$ d'acide chlorydrique 22° Be, filtre, rince au méthanol et concentre sous pression réduite à 50° C. On reprend avec 10 $cm^3$ d'eau, alcalinise avec 2,6 $cm^3$ de lessive de soude, extrait au chlorure de méthylène, lave à l'eau, sèche, filtre et concentre sous pression réduite.
On obtient 1,45 g d'huile jaune que l'on reprend par de l'éther isopropylique, filtre l'insoluble, traite au charbon actif, filtre, concentre à sec sous pression réduite à 50° C et obtient 1,24 g de produit attendu.

<u>Stade D</u> : (E) 2-butènedioate de 3,4-diméthoxy N-méthyl N-[5-(1-pyrrolidinyl) 6,7,8,9-tétrahydro 5H-benzocyclohepten-6-yl] benzèneacétamide.

On opère comme au stade C des exemples 5 et 6 à partir de 2,4 g d'acide 3,4-diméthoxyphénylacétique et de 1,9 g de produit obtenu au stade C précédent.

On obtient 2,7 g de produit sous forme de base puis 1,9 g de produit sous forme de sel avec 0,74 g d'acide fumarique après deux recristallisations dans l'éthanol.
On obtient 1,44 g de sel attendu F = 158° C.

| Analyse | C % | H % | N % |
|---|---|---|---|
| Calculé | 65,73 | 7,58 | 4,79 |
| Trouvé | 65,4 | 7,2 | 4,8 |

**Exemple 15** : (E) 2-butènedioate de (±) trans 3,4-dichloro N-méthyl N-[5-(1-pyrrolidinyl) 6,7,8,9-tétrahydro 5H-benzocyclohepten-6-yl] benzèneacétamide.

On opère comme à l'exemple précédent à partir de 2 g d'acide 3,4-dichlorophénylacétique et de 1,5 g de produit obtenu au stade C de l'exemple précédent.
On obtient 3,27 g de produit sous forme de base puis 2,9 g de sel avec 0,72 g d'acide fumarique, que l'on recristallise deux fois dans l'éthanol pour obtenir 2,6 g de sel attendu F = 292° C.

| Analyse | C % | H % | Cl % | N % |
|---|---|---|---|---|
| Calculé | 61,43 | 5,89 | 12,95 | 5,12 |
| Trouvé | 61,2 | 5,9 | 13,0 | 5,0 |

**Exemple 16** : Chlorhydrate de trans (±) 2-(3,4-dichlorophénoxy) N-méthyl N-[6-(1-pyrrolidinyl) 6,7,8,9-tétrahydro 5H-benzocyclohepten-5-yl] acétamide.

On opère comme au stade C des exemples 5 et 6 à partir de 620 mg d'acide 3,4-dichlorophénoxyacétique et de 492 mg de produit obtenu au stade B des exemples 5 et 6.
On obtient 1,1 g de produit brut que l'on cristallise dans l'éther éthylique et obtient 432 mg de produit sous forme de base transformé en 398 mg de produit sous forme de chlorhydrate recristallisé une première fois dans l'acétate d'éthyle et le méthanol et une seconde fois dans l'isopropanol et le méthanol.
On obtient 243 mg de produit attendu sous forme de chlorhydrate F = 190° C.

| Analyse | C % | H % | Cl % | N % |
|---|---|---|---|---|
| Calculé | 59,58 | 6,04 | 21,98 | 5,79 |
| Trouvé | 59,7 | 6,0 | 21,9 | 5,7 |

**Exemple 17** : Trans (±) 2-(3,4-dichlorophénoxy) N-méthyl N-[2-(1-pyrrolidinyl) 1,2,3,4-tétrahydro 1-naphtyl] acétamide et son chlorhydrate.

On opère comme indiqué au stade C de l'exemple 1 à partir de 1,4 g d'acide 3,4-dichlorophénoxyacétique et de 1,2 g de produit obtenu au stade B de l'exemple 1.
On obtient 2,8 g de produit brut que l'on purifie par chromatographie sur silice (éluant : chlorure de méthylène 95 - méthanol 05) pour obtenir 1,4 g de produit attendu sous forme de base.

On transforme le produit en chlorhydrate par addition d'une solution acide chlorhydrique/acétate d'éthyle. Après essorage, on obtient 1,1 g de produit sous forme de chlorhydrate que l'on recristallise dans le méthanol/éthylique. On obtient 850 mg de chlorhydrate F = 185° C.

| Analyse | C % | H % | N % | Cl % |
|---|---|---|---|---|
| Calculé | 58,8 | 5,79 | 5,96 | 22,04 |
| Trouvé | 58,8 | 5,9 | 5,9 | 22,4 |

**Exemples 18 et 19** : Chlorhydrate de (1-alpha, 2-béta) (±) 3,4-dichloro N,alpha-diméthyl N-[2-(1-pyrrolidinyl) 1,2,3,4-tétrahydro 1-naphtyl] benzèneacétamide.

Exemple 18 : Diastéréoisomère A.
Exemple 19 : Diastéréoisomère B.

On agite durant 30 minutes à température ambiante 1,7 g d'acide 3,4-dichloro alpha-méthyl benzène acétique et 1,3 g de 1,1' carbonyl diimidazole dans 17 $cm^3$ de tétrahydrofuranne, ajoute 1 g de produit obtenu au stade B de l'exemple 1, agite 6 heures à reflux, élimine le tétrahydrofuranne sous pression réduite, ajoute 50 $cm^3$ d'une solution saturée de bicarbonate de sodium dans 30 $cm^3$ d'eau, extrait au chlorure de méthylène, lave par une solution saturée de chlorure de sodium, sèche et concentre sous pression réduite.

On purifie le produit brut obtenu par chromatographie sur silice (éluant : chlorure de méthylène 95 - méthanol 05) et sépare 1 g de produit correspondant au diastéréoisomère A sous forme de base (exemple 18) et 1 g au diastéréoisomère B sous forme de base (exemple 19).

Obtention des chlorhydrates.

On dissout 1 g de diastéréoisomère A dans 8 $cm^3$ d'isopropanol acidifié par de l'acide chlorhydrique dans de l'acétate d'éthyle, concentre, amorce la cristallisation, ajoute 10 $cm^3$ d'acétate d'éthyle et essore.
On obtient 560 mg de produit sous forme de chlorhydrate que l'on recristallise dans l'acétate d'éthyle.
On obtient 450 mg de produit attendu F = 200° C.

| Analyse | C % | H % | N % | Cl % |
|---|---|---|---|---|
| Calculé | 61,61 | 6,25 | 5,99 | 22,73 |
| Trouvé | 61,8 | 6,4 | 5,8 | 22,8 |

Chlorhydrate du diastéréoisomère B.

On opère comme précédemment. On obtient 630 mg de produit sous forme de chlorhydrate que l'on recristallise dans l'isopropanol pour obtenir 450 mg de produit attendu F = 255° C.

| Analyse | C % | H % | N % | Cl % |
|---|---|---|---|---|
| Calculé | 61,61 | 6,25 | 5,99 | 22,73 |
| Trouvé | 61,96 | 6,5 | 5,9 | 22,9 |

**Exemple 20** : Trans (±) N-méthyl N-[2-(1-pyrrolidinyl) 1,2,3,4-tétrahydro 1-naphtyl] 4-benzo[b] thiophène acétamide.

On opère comme indiqué au stade C de l'exemple 1 à partir de 700 mg d'acide 4-thionaphtène acétique et de 700 mg de produit obtenu au stade B de l'exemple 1.
On obtient 1,8 g de produit brut que l'on chromatographie sur silice (éluant : chlorure de méthylène 9-méthanol 1) et récupère 1,2 g de produit correspondant à la fraction ayant un Rf de 0,45.

400 mg de ce produit sont repris à plusieurs reprises par 8 cm3 d'éther éthylique et évaporés à sec, à chaque fois, sous pression réduite, de façon à obtenir 350 mg de mousse incolore pulvérulente.

| Analyse | C% | H% | N% | S% |
|---|---|---|---|---|
| Calculé | 74,22 | 6,98 | 6,93 | 7,91 |
| Trouvé | 74,3 | 7,2 | 6,5 | 7,5 |

**Exemple 21** : Trans (±) 3,4-dichloro N-[7-nitro 2-(1-pyrrolidinyl) 1,2,3,4-tétrahydro-1-naphtyl] N-méthyl benzène acétamide.

Stade A : Trans (±) 7-nitro 1-(1-pyrrolidinyl) 1,2,3,4-tétrahydro-2-naphtol.

On agite à 20¤C un mélange de 4,5 g de 7-nitro 1a,2,3,7b-tétrahydronaphto [1,2-b] oxirène et 13,5 cm3 d'eau. On refroidit à 10¤C et ajoute en 15 minutes 22,5 cm3 de pyrrolidine. On agite à 20¤C pendant 4 heures, ajoute 80 cm3 d'eau, extrait au chlorure de méthylène, lave la phase organique à l'eau, sèche et évapore le solvant. On obtient 7 g de produit attendu que l'on chromatographie sur silice en éluant au mélange acétate d'éthyle-cyclohexane-triéthylamine (60-40-3). On obtient 5,43 g de produit attendu ainsi que 0,7 g de l'isomère cis correspondant. On cristallise les 5,43 g de produit trans dans un mélange éther éthylique-éther de pétrole. On obtient 4,30 g de produit attendu. F = 77¤C.
Après recristallisation dans le mélange ci-dessus, on obtient un produit fondant à 80¤C.

| Analyse : | $C_{14}H_{18}N_2O_3$ : 262,31 | | |
|---|---|---|---|
| Calculé : | C% 64,11 | H% 6,92 | N% 10,68 |
| Trouvé : | 64,3 | 6,9 | 10,7 |

Stade B : Trans (±) 7-nitro N-méthyl 2-(1-pyrrolidinyl) 1,2,3,4-tétrahydro-1-naphtalènamine.

On opère comme indiqué au stade B de l'exemple 1, à partir de 1,31 g de produit obtenu au stade A, 10 cm3 de tétrahydrofuranne, de 0,90 cm3 de triéthylamine et de 0,52 cm3 de chlorure de méthanesulfonyle dans 1,3 cm3 de tétrahydrofuranne que l'on ajoute à -20¤C. On utilise 7 cm3 de méthylamine en solution à 33% dans l'éthanol. Après 1 heure sous agitation, on évapore le solvant, ajoute 50 cm3 d'eau saturée de chlorure de sodium et 2 cm3 de soude à 32%. On extrait au chlorure de méthylène, lave à l'eau la phase organique, la sèche et évapore le solvant. On obtient 1,55 g de produit attendu que l'on cristallise dans l'isopropanol. On obtient 1,03 g de produit. F = 85¤C.

| Analyse : | $C_{15}H_{21}N_3O_2$ : 275,35 | | |
|---|---|---|---|
| Calculé : | C% 65,43 | H% 7,69 | N% 15,26 |
| Trouvé : | 65,5 | 7,8 | 15,3 |

Stade C : Trans (±) 3,4-dichloro N-[7-nitro 2-(1-pyrrolidinyl) 1,2,3,4-tétrahydro-1-naphtyl] N-méthyl benzène acétamide.

On opère comme indiqué au stade C de l'exemple 1, en utilisant 1,82 g d'acide 3,4 dichlorophényl acétique et 1,5 g de produit obtenu au stade B ci-dessus. On obtient 3 g de produit attendu que l'on cristallise dans l'éther éthylique.
On obtient 2,82 g de cristaux fondant à 133¤C. On dissout les cristaux dans un mélange de 10 cm3 de chlorure de méthylène et 50 cm3 d'isopropanol, ajoute une solution d'acide chlorhydrique dans l'éthanol jusqu'à pH≃ 2, concentre la solution et essore les cristaux. On obtient 2,26 g de produit attendu. F = 240¤C. On recristallise le produit dans l'isopropanol.

| Analyse : | $C_{23}H_{25}Cl_2N_3O_3$, HCl : 498,84 | | | |
|---|---|---|---|---|
| Calculé : | C% 55,38 | H% 5,25 | Cl % 21,32 | N% 8,42 |
| Trouvé : | 55,6 | 5,2 | 21,3 | 8,3 |

**Préparation du 7-nitro 1a,2,3,7b-tétrahydronapht [1,2-b] oxirène.**

1) 1,2-dihydro 6-nitro naphtalène.

On mélange 4 g de 7-nitro 1,2,3,4-tétrahydro-1-naphtalénol (J. Pharm. Soc. Japan. 64, (1944), p. 153),

53 cm3 de toluène et 180 mg d'acide paratoluène sulfonique. On agite 2 heures à 130¤C en distillant ≃ 20 cm3 de solvant, puis ajoute à 20¤C, 50 cm3 d'eau. On extrait à l'acétate d'éthyle, lave avec une solution aqueuse saturée de bicarbonate de sodium et avec une solution saturée de chlorure de sodium, sèche et concentre à sec. On cristallise le résidu dans l'éthanol et obtient 2,75 g de produit attendu. F ≃ 35¤C que l'on recristallise dans l'isopropanol. F = 37¤C.

| | | | | |
|---|---|---|---|---|
| Analyse | : | $C_{10}H_9NO_2$ : 175,19 | | |
| Calculé | : | C% 68,56 | H% 5,18 | N% 8,00 |
| Trouvé | : | 68,4 | 5,1 | 7,8 |

2) 7-nitro 1a,2,3,7b-tétrahydro napht [1,2-b] oxirène.

On agite à 20¤C 2,45 g de produit obtenu au stade 1, 36 cm3 de chlorure de méthylène, 47 cm3 de bicarbonate de sodium en solution 0,5N et 3,40 g d'acide métachloroperbenzoïque. On agite 5 heures à 20¤C en ajoutant après 2 heures 0,7 g de peracide et 9,30 cm3 de solution de bicarbonate. On décante, extrait au chlorure de méthylène, lave à l'eau, sèche et évapore le solvant. On obtient après cristallisation dans l'éther 1,6 g de produit attendu. F = 67¤C. Après recristallisation dans l'isopropanol le produit fond à 73¤C.

| | | | | |
|---|---|---|---|---|
| Analyse | : | $C_{10}H_9NO_3$ : 191,19 | | |
| Calculé | : | C% 62,82 | H% 4,75 | N% 7,33 |
| Trouvé | : | 62,6 | 4,7 | 7,2 |

## Exemples de composition pharmaceutique.

### Exemple 22 :

On a préparé des comprimés répondant à la formule suivante :

- Produit de l'exemple 20 200 mg
- Excipient q.s.p. 800 mg

(Détail de l'excipient : lactose, talc, amidon, stéarate de magnésium).

### Exemple 23 :

On a préparé un soluté injectable (voie intra-musculaire) répondant à la formule suivante :

- Produit de l'exemple 2 50 mg
- Solvant stérile q.s.p. 5 cm3

## ETUDE PHARMACOLOGIQUE

1) Liaison au récepteur opiacé K in vitro.

On utilise des culots membranaires conservés à -30¤C (éventuellement pendant 30 jours) et préparés à partir de cervelets de cobayes.

Ces culots sont remis en suspension dans le tampon Tris pH 7,7. On répartit les fractions de 2 cm3 dans les tubes à hémolyse et ajoute de la $^3H$ éthylkétocyclazocine 1nM et le produit à étudier.

On détermine :

- la quantité maximale de radioactivité fixée en présence de $^3H$ éthylkétocyclazocine seule,
- la quantité de radioactivité liée de façon non spécifique, en présence du ligand tritié et d'un excès ($10^{-5}$M) de produit de référence connu sous le nom U-50488 H,
- le déplacement de liaison spécifique produit par une dose de 5 x $10^{-6}$M de produit à tester.

Ces mesures sont effectuées en triplicate. Pour cela on incube à 25¤C pendant 40 minutes les aliquotes de 2 cm3. Après retour à 0¤C, 5 minutes, à la fin de l'incubation, on filtre sous vide sur filtres Whatman GF/C, rince au tampon Tris pH 7,7 et compte la radioactivité en présence de scintillant Triton®.

Lorsque le produit testé déplace de plus de 50% la radioactivité fixée spécifiquement, on détermine à l'aide

d'une gamme de 7 doses sa concentration inhibitrice 50% ($CI_{50}$).

On exprime alors le résultat de la concentration inhibitrice 50% en nM. La $CI_{50}$ est donc la concentration de produit étudié, exprimée en nM, nécessaire pour déplacer 50% de la radioactivité spécifique fixée sur le récepteur étudié (LAHTI et al. 1982, Life Sci. 31, 2257).

| Résultats | $CI_{50}$ en nM |
|---|---|
| Produit de l'exemple 2 | 12 |
| Produit de l'exemple 3 | 11 |
| Produit de l'exemple 20 | 3,3 |

2) Mesure de l'activité diurétique.

Des rats mâles, de souche Sprague Dawley et de poids 180-200 g, sont mis à jeun 17 heures avant l'essai, tout en recevant de l'eau ad libitum.

Des lots de 8 animaux sont constitués par dose testée. Les rats reçoivent le produit à tester ou son véhicule par voie sous-cutanée.

Le volume urinaire est mesuré chaque heure pendant les 2 heures qui suivent l'administration du produit. A la fin de cette période, les urines sont recueillies et l'activité du produit est exprimée en pourcentage de variation calculé sur le volume urinaire correspondant à la période $t_{1h}$-$t_{2h}$.

Les produits de la demande démontrent une activité notable sur ce test.

## Revendications

1. Composés de formule (I) :

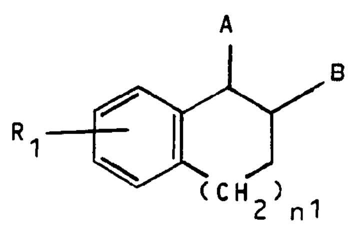

(I)

dans laquelle $R_1$ représente un atome d'hydrogène, un atome d'halogène, un radical alkyle ou alcoxy renfermant de 1 à 5 atomes de carbone, nitro, amino, monoalkylamino ou dialkylamino, $n_1$ représente 1 ou 2, et A et B sont en configuration trans et sont tels que l'un des substituants A ou B est choisi dans le groupe de formule :

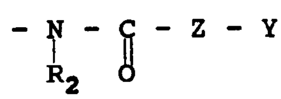

$R_2$ étant un atome d'hydrogène ou un radical alkyle renfermant de 1 à 5 atomes de carbone, Z une chaîne alkylène linéaire $-(CH_2)_{n_2}-$, $n_2$ étant un nombre entier pouvant varier entre O et 5 ou une chaîne alkylène ramifiée renfermant de 2 à 8 atomes de carbone ou un groupement $-CH_2-O-$, Y représente un radical phényle éventuellement substitué par un ou plusieurs radicaux alkyle ou alcoxy renfermant de 1 à 5 atomes de carbone, atomes d'halogène, radicaux trifluorométhyle, nitro, amino, monoalkyle ou dialkylamino ; un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, et l'autre des substituants A ou B est choisi dans le groupe de formule :

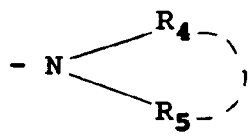

$R_4$ et $R_5$, identiques ou différents, représentant un atome d'hydrogène ou un radical alkyle renfermant de 1 à 5 atomes de carbone ou $R_4$ et $R_5$ forment ensemble avec l'atome d'azote auquel ils sont liés un radical pyrrolidinyle, piperazinyle, piperidinyle, morpholinyle, et à la condition que
ou bien A représente un groupe de formule

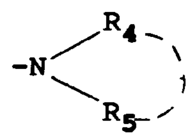

dans lequel $R_4$ et $R_5$ ont la signification précédente et B représente un groupe de formule

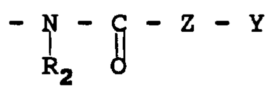

dans lequel $R_2$, Z et Y ont la signification précédente ;
ou bien Z représente $-(CH_2)n_2-$ dans lequel $n_2$ est 0, 2, 3, 4 ou 5, ou une chaîne alkylène ramifiée renfermant de 2 à 8 atomes de carbone ou un groupement $-CH_2-O-$ ;
ou bien Y représente un radical phényle substitué par un ou plusieurs radicaux alkyle renfermant de 1 à 5 atomes de carbone, alkoxy renfermant de 2 à 5 atomes de carbone, amino, monoalkyle ou dialkylamino ou Y représente un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, à l'exception du benzothiophène non substitué ;
ou bien $R_1$ représente un radical nitro ;
ou bien $R_2$ représente un radical alkyle renfermant 4 ou 5 atomes de carbone ;
ou bien $R_1$ représente un atome d'hydrogène, $n_1$ représente 1, A représente le groupement

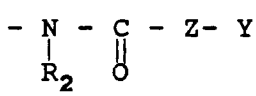

dans lequel $R_2$ est $CH_3$, Z est $CH_2$ et Y est un radical 3,4-diméthoxy-phényle, 4-nitrophényle ou 4-benzothiényle et B représente le radical pyrrolidinyle ;
ou bien $R_1$ représente un atome d'hydrogène, $n_1$ représente 2, A représente le groupement

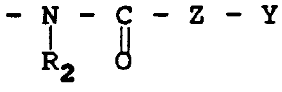

dans lequel $R_2$ est $CH_3$, Z est $CH_2$ et Y est un radical 3,4-diméthoxy-phényle, 3,4-dichlorophényle, 4-trifluorométhylphényle, 4-nitrophényle ou 4-benzothiényle et B représente le radical pyrrolidinyle ; lesdits composés de formule (I) pouvant être dans toutes les formes énantiomères et diastéréoisomères possibles et sous forme de sels d'addition avec les acides.

**2.** Composés de formule (I) caractérisés en ce que dans le groupe

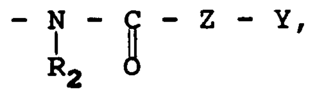

$R_2$ est un radical méthyle ou éthyle, Z un groupe $(CH_2)_{n2}$ - dans lequel $n_2$ est 0 ou 1,

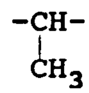

ou $-CH_2$-O-, et en ce que dans le groupe

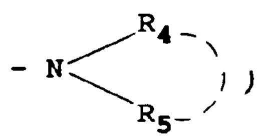

$R_4$ et $R_5$ forment avec l'atome d'azote auquel ils sont liés l'hétérocycle pyrrolidine, ainsi que leurs sels d'addition avec les acides.

**3.** Composés de formule (I) caractérisés en ce que dans le groupe

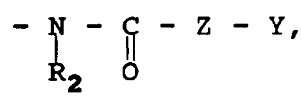

Y représente un radical phényle substitué éventuellement par un ou deux radicaux identiques choisis parmi les radicaux méthyle, éthyle, méthoxy et éthoxy, les atomes de chlore ou de brome, les radicaux trifluorométhyle et nitro, ou Y représente un radical naphtyle ou benzo[b] thiophène, ainsi que leurs sels d'addition avec les acides.

**4.** Composés de formule (I) :

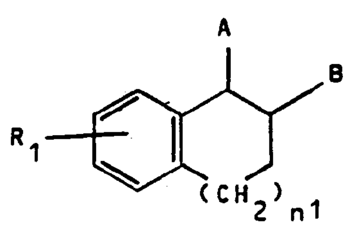

(I)

dans laquelle $R_1$ représente un atome d'hydrogène, un atome d'halogène, un radical alkyle ou alcoxy renfermant de 1 à 5 atomes de carbone, nitro, amino, monoalkylamino ou dialkylamino, $n_1$ représente 1 ou 2, et A et B sont en configuration trans et sont tels que l'un des substituants A ou B représente un radical de formule :

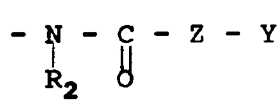

$R_2$ représente un atome d'hydrogène ou un radical alkyle renfermant de 1 à 5 atomes de carbone, Z représente une chaîne alkyleène linéaire $-(CH_2)_{n_2}-$ , $n_2$ étant un nombre entier pouvant varier entre O et 5 ou une chaîne alkylène ramifiée renfermant de 2 à 8 atomes de carbone ou un groupement $-CH_2$-O-,

Y représente un radical phényle éventuellement substitué par un ou plusieurs radicaux alkyle ou alcoxy renfermant de 1 à 5 atomes de carbone, atomes d'halogène, radicaux trifluorométhyle, nitro, amino, monoalkyle ou dialkylamino; un radical naphtyle, un radical indényle, un radical thiazolyl, pyridinyle, oxazolyle, isoxazolyle, imidazolyle ou thiényle ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents choisis parmi les radicaux méthyle ou éthyle,méthoxy ou éthoxy, un atome de chlore, brome ou fluor, trifluorométhyle, nitro ou phényle, et l'autre des substituants A ou B représente un radical de formule :

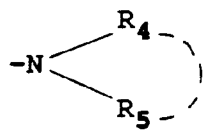

$R_4$ et $R_5$, identiques ou différents, représentent un atome d'hydrogène ou un radical alkyle renfermant de 1 à 5 atomes de carbone ou $R_4$ et $R_5$ forment ensemble avec l'atome d'azote auquel ils sont liés un radical pyrrolidinyle, piperazinyle, piperidinyle, morpholinyle, et à la condition que
ou bien A représente un groupe de formule

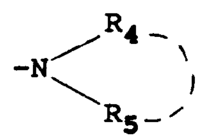

dans lequel $R_4$ et $R_5$ ont la signification précédente et B représente un groupe de formule

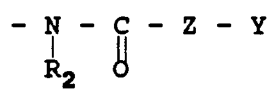

dans lequel $R_2$, Z et Y ont la signification précédente ;
ou bien Z représente $-(CH_2)n_2-$ dans lequel $n_2$ est 0, 2, 3, 4 ou 5, ou une chaîne alkylène ramifiée renfermant de 2 à 8 atomes de carbone ou un groupement $-CH_2$-O- ;
ou bien Y représente un radical phényle substitué par un ou plusieurs radicaux alkyle renfermant de 1 à 5 atomes de carbone, alkoxy renfermant de 2 à 5 atomes de carbone, amino, monoalkyle ou dialkylamino ou Y représente un radical naphtyle, un radical indényle, un radical thiazolyle, pyridinyle, oxazolyle, isoxazolyle, imidazolyle ou thiényle, ou un radical indolyle, quinolyle, benzofurannyle, benzo[b]thiényle, benzimidazolyle, benzoxazolyle ou benzothiazolyle, tous ces radicaux étant éventuellement substitués par un ou plusieurs radicaux identiques ou différents, choisis parmi les radicaux méthyle ou éthyle, méthoxy ou éthoxy, un atome de chlore, brome ou fluor, trifluorométhyle, nitro ou phényle, à l'exception du benzothiophène non substitué ;
ou bien $R_1$ représente un radical nitro ;
ou bien $R_2$ représente un radical alkyle renfermant 4 ou 5 atomes de carbone ;
lesdits composés de formule (I) pouvant être dans toutes les formes énantiomères et diastéréoisomères possibles et sous forme de sels d'addition avec les acides.

**5.** Les composés de formule (I), telle que définie à la revendication 1, dans laquelle :
ou bien $R_1$ représente un atome d'hydrogène, $n_1$ représente 1, A représente le groupement

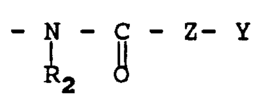

dans lequel $R_2$ est $CH_3$, Z est $CH_2$ et Y est un radical 3,4-diméthoxy-phényle, 4-nitrophényle ou 4-benzothiényle et B représente le radical pyrrolidinyle ;
ou bien $R_1$ représente un atome d'hydrogène, $n_1$ représente 2, A représente le groupement

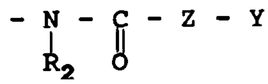

dans lequel $R_2$ est $CH_3$, Z est $CH_2$ et Y est un radical 3,4-diméthoxy-phényle, 3,4-dichlorophényle, 4-trifluorométhylphényle, 4-nitrophényle ou 4-benzothiényle et B représente le radical pyrrolidinyle ; ainsi que leurs sels d'addition avec les acides.

6. - Le trans (±) 4-nitro N-méthyl N-[2-(1-pyrrolidinyl) 1,2,3,4-tétrahydro 1-naphtyl] benzèneacétamide ;
- Le trans (±) N-méthyl N-[2-(1-pyrrolidinyl) 1,2,3,4-tétrahydro 1-naphtyl] 1-naphtalèneacétamide ;
- Le trans (±) N-méthyl N-[2-(1-pyrrolidinyl) 1,2,3,4-tétrahydro 1-naphtyl] 4-benzo[b]thiophèneacétamide ; ainsi que leurs sels d'addition avec les acides.

7. Procédé de préparation des produits de formule (I) telle que définie à la revendication 1, caractérisé en ce que l'on condense le produit de formule (II) :

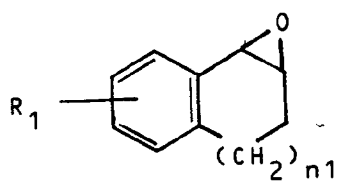

(II)

$R_1$ et $n_1$ ayant les significations données précédemment,
- soit avec une amine de formule (III) :

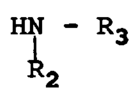

(III)

$R_2$ ayant la signification donnée à la revendication 1 et $R_3$ étant un groupement protecteur de la fonction amine pour obtenir un produit de formule (IV) :

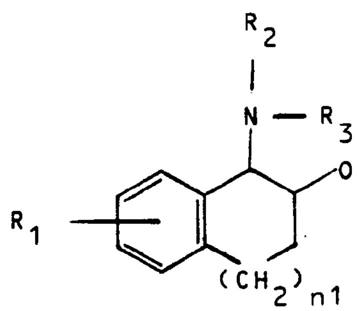

(IV)

dont on active la fonction hydroxyle et que l'on condense avec une amine

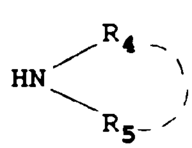

dans laquelle $R_4$ et $R_5$ ont les significations indiquées à la revendication 1 pour obtenir un produit de formule (V) :

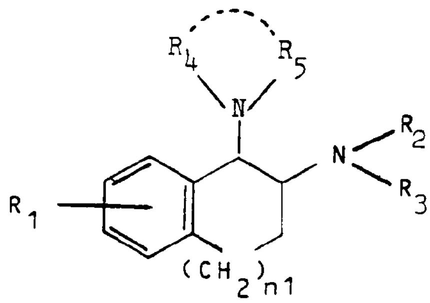

(V)

dont on élimine le groupement protecteur $R_3$ de la fonction amine pour obtenir un produit de formule (VI) :

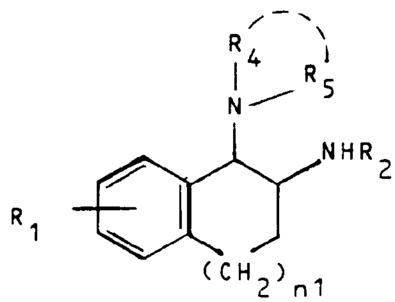

(VI)

que l'on traite avec un acide de formule (VII) ou un dérivé fonctionnel de cet acide :

Y-Z-COOH (VII)

dans laquelle Y et Z ont les significations indiquées à la revendication 1 pour obtenir un produit de formule (I) dans laquelle Y représente

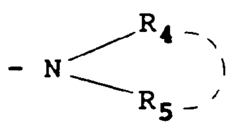 et B le groupe 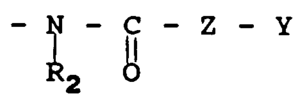

- soit avec une amine de formule

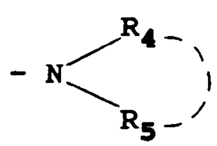

pour obtenir un produit de formule (VIII) :

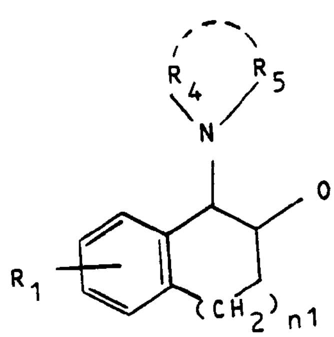

(VIII)

dont on active la fonction hydroxyle et que l'on traite avec une amine de formule (IX) :

$NH_2R_2$ (IX)

dans laquelle $R_2$ a la signification précédente pour obtenir un produit de formule (X) :

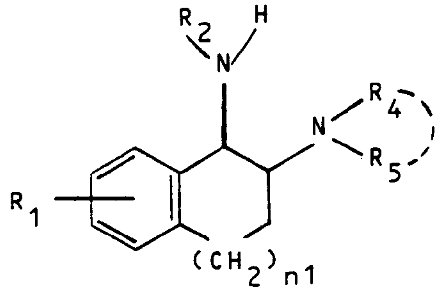

(X)

que l'on condense avec un acide de formule (VII) ou un dérivé fonctionnel de cet acide :

Y-Z-COOH (VII)

dans laquelle Z et Y ont les significations précédentes pour obtenir un produit de formule (I) dans laquelle A représente le groupe

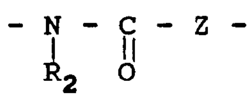 et B 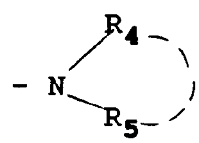 ,

lesdits composés de formule (I) pouvant être dédoublés pour obtenir les formes optiquement actives et traite, si désiré, avec un acide minéral ou organique, pour obtenir les sels.

**8.** A titre de médicaments, les produits de formule (I), tels que définis à l'une quelconque des revendications 1 à 4, ainsi que leurs sels d'addition avec les acides pharmaceutiquement acceptables.

**9.** A titre de médicaments, les produits de formule (I) tels que définis à la revendication 5, ainsi que leurs sels d'addition avec les acides pharmaceutiquement acceptables.

**10.** A titre de médicaments, les produits de la revendication 6.

**11.** Les compositions pharmaceutiques contenant à titre de principe actif, l'un au moins des médicaments tels que définis par les revendications 8, 9 ou 10.

**12.** A titre de produits intermédiaires, les produits de formules (V), (VI) et (X) telles que définies à la revendication 7 et telles que pour les produits de formules (V) et (VI), l'une au moins des conditions suivantes est remplie :

- $R_4$ et $R_5$ ne représentent pas un atome d'hydrogène ;
- $R_1$ représente un radical nitro ;
- $R_2$ représente un radical alkyle ayant 4 ou 5 atomes de carbone et pour les produits de formule (X), l'une au moins des conditions suivantes est remplie :
  - $R_1$ représente un radical nitro ;
  - $R_2$ représente un radical alkyle ayant 4 ou 5 atomes de carbone.

**Patentansprüche**

**1.** Verbindungen der Formel (I)

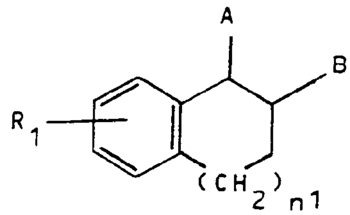

(I)

worin $R_1$ für ein Wasserstoffatom, ein Halogenatom, einen Alkyl- oder Alkoxyrest mit 1 bis 5 Kohlenstoffatomen, Nitro, Amino, Monoalkylamino oder Dialkylamino steht, $n_1$ für 1 oder 2 steht und A und B sich in trans-Konfiguration befinden und derart sind, daß einer der Substituenten A oder B ausgewählt ist unter der Gruppe der Formel

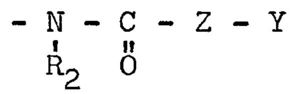

wobei $R_2$ ein Wasserstoffatom oder einen Alkylrest mit 1 bis 5 Kohlenstoffatomen bedeutet, Z eine lineare Alkylenkette - $(CH_2)_{n_2}$, worin $n_2$ eine ganze Zahl ist, die zwischen 0 und 5 variieren kann, oder eine verzweigte Alkylenkette mit 2 bis 8 Kohlenstoffatomen oder eine Gruppe -$CH_2$-O- wiedergibt, Y einen Phenylrest, gegebenenfalls substituiert durch einen oder mehrere Alkyl- oder Alkoxyreste mit 1 bis 5 Kohlenstoffatomen, Halogenatome, Trifluormethyl-, Nitro-, Amino-, Monoalkyl- oder Dialkylamino-Reste; einen Naphthylrest, einen Indenylrest, einen Thiazolyl-, Pyridinyl-, Oxazolyl-, Isoxazolyl-, Imidazolyl- oder Thienylrest oder einen Indolyl-, Chinolyl-, Benzofuranyl-, Benzo[b]thienyl-, Benzimidazolyl-, Benzoxazolyl- oder Benzothiazolyl-Rest bedeutet, wobei alle diese Reste gegebenenfalls substituiert sind durch einen oder mehrere identische oder voneinander verschiedene Reste, ausgewählt unter den Methyl- oder Ethyl-, Methoxy-, oder Ethoxyresten, einem Chlor-, Brom- oder Fluoratom, Trifluormethyl, Nitro oder Phenyl, und der andere der Substituenten A oder B ausgewählt ist unter der Gruppe der Formel

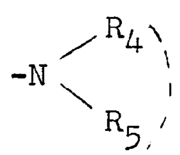

wobei $R_4$ und $R_5$, die identisch oder voneinander verschieden sind, ein Wasserstoffatom oder einen Alkylrest mit 1 bis 5 Kohlenstoffatomen bedeuten oder $R_4$ und $R_5$ gemeinsam mit dem Stickstoffatom, an das sie gebunden sind, einen Pyrrolidinyl-, Piperazinyl-, Piperidinyl oder Morpholinylrest bilden, mit der Maßgabe, daß
entweder A eine Gruppe der Formel

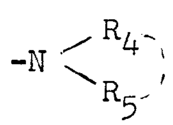

bedeutet, worin $R_4$ und $R_5$ die vorstehende Bedeutung besitzen und B für eine Gruppe der Formel

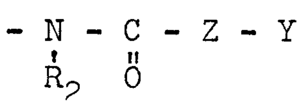

steht, worin $R_2$, Z und Y die vorstehende Bedeutung besitzen;
oder Z für - $(CH_2)_{n_2}$, worin $n_2$ 0, 2, 3, 4 oder 5 ist, oder eine verzweigte Alkylenkette mit 2 bis 8 Kohlenstoffatomen oder eine Gruppe -$CH_2$-O- steht;

oder Y einen Phenylrest wiedergibt, der substituiert ist durch einen oder mehrere Alkylreste mit 1 bis 5 Kohlenstoffatomen, Alkoxyreste mit 2 bis 5 Kohlenstoffatomen, Amino, Monoalkyl oder Dialkylamino oder Y einen Naphthylrest, einen Indenylrest, einen Thiazolyl-, Pyridinyl-,Oxazolyl-, Isoxazolyl-, Imidazolyl- oder Thienylrest oder einen Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls substituiert sind durch einen oder mehrere identische oder voneinander verschiedene Reste, ausgewählt unter den Methyl- oder Ethyl-, Methoxy- oder Ethoxyresten, einem Chlor-, Brom- oder Fluoratom, Trifluormethyl, Nitro oder Phenyl, mit der Ausnahme von unsubstituiertem Benzothiophen;
oder $R_1$ für eine Nitrogruppe steht;
oder $R_2$ einen Alkylrest mit 4 oder 5 Kohlenstoffatomen bedeutet;
oder $R_1$ ein Wasserstoffatom bedeutet, $n_1$ für 1 steht, A die Gruppe

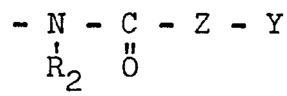

wiedergibt, worin $R_2$ für $CH_3$ steht, Z für $CH_2$ steht und Y ein 3,4-Dimethoxyphenyl-, 4-Nitrophenyl- oder 4-Benzothienylrest ist und B für den pyrrolidinylrest steht;
oder $R_1$ ein Wasserstoffatom bedeutet, $n_1$ für 2 steht, A die Gruppe

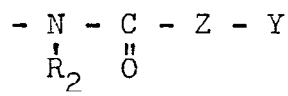

bedeutet, worin $R_2$ für $CH_3$ steht, Z für $CH_2$ steht und Y ein 3,4-Dimethoxyphenyl-, 3,4-Dichlorphenyl-, 4-Trifluormethylphenyl-, 4-Nitrophenyl- oder 4-Benzothienylrest ist, und B den Pyrrolidinylrest darstellt; wobei alle diese Verbindungen der Formel (I) in sämtlichen ihrer möglichen enantiomeren und diastereomeren Formen und in Form der Additionssalze mit Säuren vorliegen können.

**2.** Verbindungen der Formel (I), dadurch gekennzeichnet, daß in der Gruppe

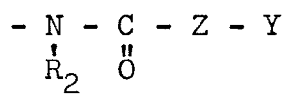

$R_2$ ein Methyl- oder Ethylrest ist, Z eine Gruppe $(CH_2)_{n_2}$, worin $n_2$ für 0 oder 1 steht,

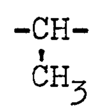

oder $-CH_2-O-$ ist, und daß in der Gruppe

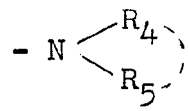

$R_4$ und $R_5$ mit dem Stickstoffatom, an das sie gebunden sind, einen Pyrrolidin-Heterocyclus bilden, sowie deren Additionssalze mit Säuren.

**3.** Verbindungen der Formel (I), dadurch gekennzeichnet, daß in der Gruppe

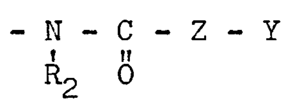

Y für einen Phenylrest steht, der gegebenenfalls durch einen oder zwei identische Reste substituiert ist, ausgewählt unter den Methyl-, Ethyl-, Methoxy- und Ethoxyresten, den Chlor- oder Bromatomen, den Trifluormethyl und Nitroresten, oder Y einen Naphthyl- oder Benzo[b]thiophenrest bedeutet, sowie deren Additionssalze mit Säuren.

**4.** Verbindungen der Formel (I)

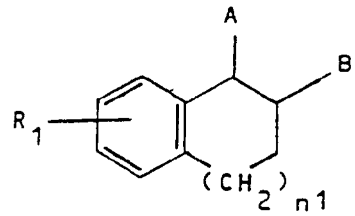

(I)

worin $R_1$ ein Wasserstoffatom, ein Halogenatom, einen Alkyl- oder Alkoxyrest mit 1 bis 5 Kohlenstoffatomen, Nitro, Amino, Monoalkylamino oder Dialkylamino bedeutet, $n_1$ für 1 oder 2 steht, und A und B sich in trans-Konfiguration befinden und derart sind, daß einer der Substituenten A oder B einen Rest der Formel

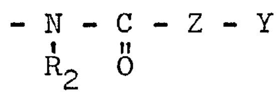

wiedergibt, wobei $R_2$ ein Wasserstoffatom oder einen Alkylrest mit 1 bis 5 Kohlenstoffatomen bedeutet, Z eine lineare Alkylenkette - $(CH_2)_{n_2}$, wobei $n_2$ eine ganze Zahl, die zwischen 0 und 5 variieren kann, bedeutet, oder eine verzweigte Alkylenkette mit 2 bis 8 Kohlenstoffatomen oder eine Gruppe $-CH_2-O-$ wiedergibt, Y einen Phenylrest, gegebenenfalls substituiert durch einen oder mehrere Alkyl- oder Alkoxyreste mit 1 bis 5 Kohlenstoffatomen, Halogenatome, Trifluormethylreste, Nitro, Amino, Monoalkyl oder Dialkylamino; einen Naphthylrest, einen Indenylrest, einen Thiazolyl-, Pyridinyl-, Oxazolyl-, Isoxazolyl-, Imidazolyl- oder Thienylrest oder einen Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls substituiert sind durch einen oder mehrere identische oder voneinander verschiedene Reste, ausgewählt unter den Methyl- oder Ethyl-, Methoxy- oder Ethoxyresten, einem Chlor-, Brom- oder Fluor- atom, Trifluormethyl, Nitro oder Phenyl, und der andere der Substituenten A oder B einen Rest der Formel

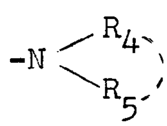

wiedergibt, wobei $R_4$ und $R_5$, die identisch oder voneinander verschieden sind, ein Wasserstoffatom oder einen Alkylrest mit 1 bis 5 Kohlenstoffatomen bedeuten oder $R_4$ und $R_5$ gemeinsam mit dem Stickstoffatom, an das sie gebunden sind, einen Pyrrolidinyl-, Piperazinyl-, Piperidinyl oder Morpholinylrest bilden, mit der Maßgabe, daß
entweder A eine Gruppe der Formel

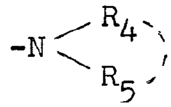

bedeutet, worin $R_4$ und $R_5$ die vorstehende Bedeutung besitzen und B eine Gruppe der Formel

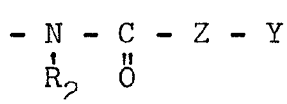

darstellt, worin $R_2$, Z und Y die vorstehende Bedeutung besitzen;
<u>oder</u> Z für $-(CH_2)_{n_2}-$, worin $n_2$ 0, 2, 3, 4 oder 5 ist, oder eine verzweigte Alkylenkette mit 2 bis 8 Kohlenstoffatomen oder einen Gruppe $-CH_2-O-$ steht;
<u>oder</u> Y einen Phenylrest, substituiert durch einen oder mehrere Alkylreste 1 bis 5 Kohlenstoffatomen, Alkoxyreste mit 2 bis 5 Kohlenstoffatomen, Amino, Monoalkyl oder Dialkylamino, bedeutet oder Y einen Naphthylrest, einen Indenylrest, einen Thiazolyl-, Pyridinyl-, Oxazolyl-, Isoxazolyl-Imidazolyl- oder Thienylrest oder einen Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls substituiert sind durch einen oder mehrere identische oder voneinander verschiedene Reste, ausgewählt unter den Methyl- oder Ethyl-, Methoxy- oder Ethoxyresten, einem Chlor-, Brom- oder Fluoratom, Trifluormethyl, Nitro oder Phenyl, mit Ausnahme von nichtsubstituiertem Benzothiophen;
<u>oder</u> $R_1$ eine Nitrogruppe bedeutet;
<u>oder</u> $R_2$ einen Alkylrest mit 4 oder 5 Kohlenstoffatomen wiedergibt;
wobei diese Verbindungen der Formel (I) in sämtlichen ihrer möglichen enantiomeren und diasteromeren Formen und in Form der Additionssalze mit Säuren vorliegen können.

5. Verbindungen der Formel (I), wie in Anspruch 1 definiert, worin
<u>entweder</u> $R_1$ ein Wasserstoffatom bedeutet, $n_1$ für 1 steht, A die Gruppe

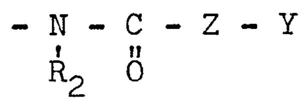

darstellt, worin $R_2$ für $CH_3$ steht, Z für $CH_2$ steht und Y ein 3,4-Dimethoxyphenyl-, 4-Nitrophenyl- oder 4-Benzothienylrest ist und B für den Pyrrolidinylrest steht;
<u>oder</u> $R_1$ ein Wasserstoffatom bedeutet, $n_1$ für 2 steht, A die Gruppe

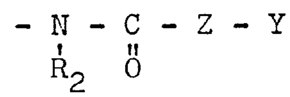

wiedergibt, worin $R_2$ für $CH_3$ steht, Z für $CH_2$ steht und Y ein 3,4-Dimethoxyphenyl-, 3,4-Dichlorphenyl-, 4-Trifluormethylphenyl-, 4-Nitrophenyl- oder 4-Benzothienylrest ist und B für den Pyrrolidinylrest steht;
sowie deren Additionssalze mit Säuren.

6. Trans-(±)-4-nitro-N-methyl-N-[2-(1-pyrrolidinyl)-1,2,3,4-tetrahydro-1-naphthyl]-benzolacetamid;
trans-(±)-N-Methyl-N-[2-(1-pyrrolidinyl)-1,2,3,4-tetrahydro-1-naphthyl]-1-naphthalinacetamid;
trans-(±)-N-Methyl-N-[2-(1-pyrrolidinyl)-1,2,3,4-tetrahydro-1-naphthyl]-4-benzo[b]thiophenacetamid;
sowie deren Additionssalze mit Säuren.

7. Verfahren zur Herstellung der Produkte der Formel (I), wie in Anspruch 1 definiert, dadurch gekennzeichnet, daß man das Produkt der Formel (II)

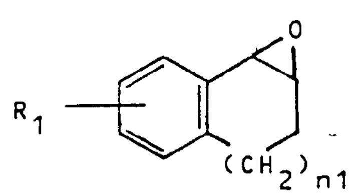

(II)

worin $R_1$ und $n_1$ die vorstehend angegebenen Bedeutungen besitzen,
- entweder mit einem Amin der Formel (III)

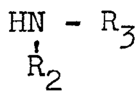 (III)

worin $R_2$ die in Anspruch 1 angegebene Bedeutung besitzt und $R_3$ eine Schutzgruppe für die Aminfunktion ist, kondensiert, um zu einem Produkt der Formel IV

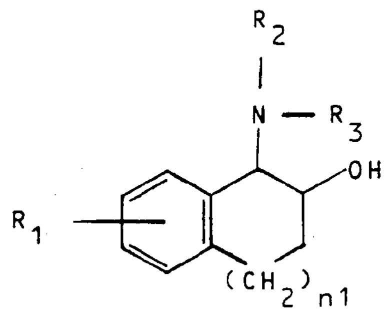 (IV)

zu gelangen, dessen Hydroxylfunktion man aktiviert und welches man mit einem Amin

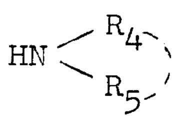,

worin $R_4$ und $R_5$ die in Anspruch 1 angegebenen Bedeutungen besitzen, kondensiert, um zu einem Produkt der Formel (V)

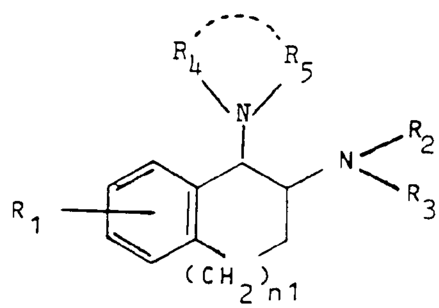 (V)

zu gelangen, dessen Schutzgruppe $R_3$ der Aminfunktion man entfernt, um zu einem Produkt der Formel (VI)

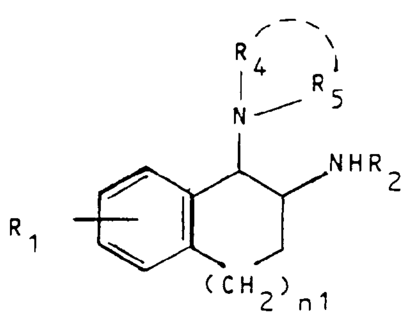 (VI)

zu gelangen, welches man mit einer Säure der Formel (VII) oder einem funktionellen Derivat dieser Säure

$$Y - Z - COOH \qquad (VII)$$

worin Y und Z die in Anspruch 1 angegebenen Bedeutungen besitzen, behandelt, um zu einem Produkt der Formel (I) zu gelangen, worin A für

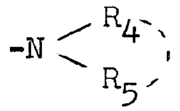

steht und B die Gruppe

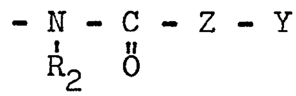

wiedergibt,

- oder mit einem Amin der Formel

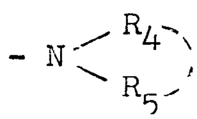

kondensiert, um zu einem Produkt der Formel (VIII)

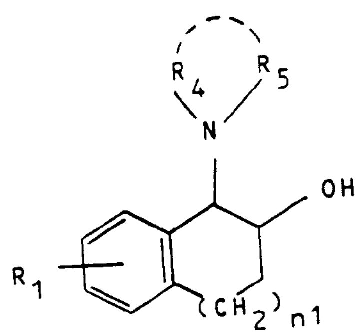

(VIII)

zu gelangen, dessen Hydroxylfunktion man aktiviert und welches man mit einem Amin der Formel (IX)

$NH_2R_2$ (IX)

worin $R_2$ die vorstehende Bedeutung besitzt, behandelt, um zu einem Produkt der Formel (X)

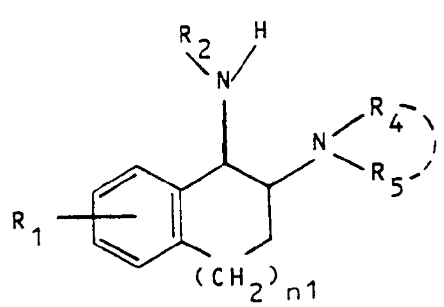

(X)

zu gelangen, welches man mit einer Säure der Formel (VII) oder einem funktionellen Derivat dieser Säure

Y-Z-COOH (VII)

worin Z und Y die vorstehenden Bedeutungen besitzen, behandelt, um ein Produkt der Formel (I) zu erhalten, worin A für die Gruppe

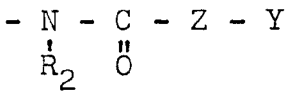 und B für 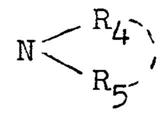

steht, wobei diese Verbindungen der Formel (I) getrennt werden können, um die optisch aktiven Formen zu erhalten, und gewünschtenfalls mit einer Mineral- oder organischen Säure behandelt, um die Salze zu erhalten.

8. Als Arzneimittel die Produkte der Formel (I), wie in einem der Ansprüche 1 bis 4 definiert, sowie deren Additionssalze mit pharmazeutisch verträglichen Säuren.

9. Als Arzneimittel die Produkte der Formel (I), wie in Anspruch 5 definiert, sowie deren Additionssalze mit pharmazeutisch verträglichen Säuren.

10. Als Arzneimittel die Produkte gemäß Anspruch 6.

11. Pharmazeutische Zusammensetzungen, enthaltend als Wirkstoff zumindest eines der Arzneimittel, wie in den Ansprüchen 8, 9 oder 10 definiert.

12. Als Zwischenprodukte die Produkte der Formeln (V), (VI) und (X), wie in Anspruch 7 definiert und derart, daß für die Produkte der Formeln (V) und (VI) zumindest eine der folgenden Bedingungen erfüllt ist:
   - $R_4$ und $R_5$ bedeuten kein Wasserstoffatom;
   - $R_1$ bedeutet eine Nitrogruppe;
   - $R_2$ bedeutet einen Alkylrest mit 4 oder 5 Kohlenstoffatomen, und für die Produkte der Formel (X) zumindest eine der folgenden Bedingungen erfüllt ist:
     - $R_1$ bedeutet eine Nitrogruppe;
     - $R_2$ bedeutet einen Alkylrest mit 4 oder 5 Kohlenstoffatomen.

## Claims

1. Compounds of formula (I):

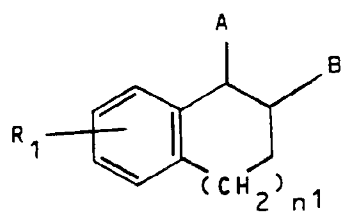

(I)

in which $R_1$ represents a hydrogen atom, a halogen atom, the following radicals: alkyl or alkoxy containing 1 to 5 carbon atoms, nitro, amino, monoalkylamino or dialkylamino, $n_1$ represents 1 or 2, and A and B are of trans configuration and are such that one of the substituents A or B is chosen from the group of formula:

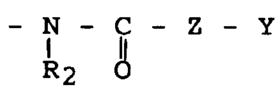

$R_2$ being a hydrogen atom or an alkyl radical containing 1 to 5 carbon atoms, Z a linear alkylene chain -$(CH_2)_{n2}$-, $n_2$ being an integer which can vary between 0 and 5 or a branched alkylene chain containing 2 to 8 carbon atoms or a -$CH_2$-O- group, Y represents a phenyl radical optionally substituted by one or more alkyl or alkoxy radicals containing 1 to 5 carbon atoms, halogen atoms, trifluoromethyl, nitro, amino, monoalkyl or dialkylamino radicals; a naphthyl radical, an indenyl radical, a thiazolyl, pyridinyl, oxazolyl, isoxazolyl, imidazolyl or thienyl radical, or one of the following radicals: indolyl, quinolyl, benzofurannyl, benzo[b]thienyl, benzimidazolyl, benzoxazolyl or benzothiazolyl, all these radicals being optionally substituted by one or more identical or different radicals, chosen from methyl or ethyl, methoxy or ethoxy radicals,

a chlorine, bromine or fluorine atom, trifluoromethyl, nitro or phenyl radicals, and the other substituent A or B is chosen from the group of formula:

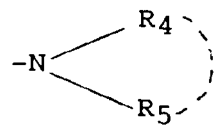

$R_4$ and $R_5$, identical or different, representing a hydrogen atom or an alkyl radical containing 1 to 5 carbon atoms or $R_4$ and $R_5$ form together with the nitrogen atom to which they are linked a pyrrolidinyl, piperazinyl, piperidinyl, morpholinyl radical, and on condition that
either A represents a group of formula

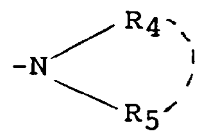

in which $R_4$ and $R_5$ have the previous meaning and B represents a group of formula

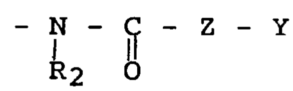

in which $R_2$, Z and Y have the previous meaning;
or Z represents $-(CH_2)n_2-$ in which $n_2$ is 0, 2, 3, 4 or 5, or a branched alkylene chain containing 2 to 8 carbon atoms or a $-CH_2-O-$ group;
or Y represents a phenyl radical substituted by one or more of the following radicals: alkyl containing 1 to 5 carbon atoms, alkoxy containing 2 to 5 carbon atoms, amino, monoalkyl or dialkylamino or Y represents a naphthyl radical, an indenyl radical, a thiazolyl, pyridinyl, oxazolyl, isoxazolyl, imidazolyl or thienyl radical, or one of the following radicals: indolyl, quinolyl, benzofurannyl, benzo[b]thienyl, benzimidazolyl, benzoxazolyl or benzothiazolyl, all these radicals being optionally substituted by one or more identical or different radicals, chosen from methyl or ethyl, methoxy or ethoxy radicals, a chlorine, bromine or fluorine atom, trifluoromethyl, nitro or phenyl radicals, with the exception of the non-substituted benzothiophene;
or $R_1$ represents a nitro radical;
or $R_2$ represents an alkyl radical containing 4 or 5 carbon atoms;
or $R_1$ represents a hydrogen atom, $n_1$ represents 1, A represents the group

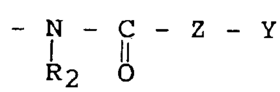

in which $R_2$ is $CH_3$, Z is $CH_2$ and Y is a 3,4-dimethoxy-phenyl, 4-nitrophenyl or 4-benzothienyl radical and B represents the pyrrolidinyl radical;
or $R_1$ represents a hydrogen atom, $n_1$ represents 2, A represents the group

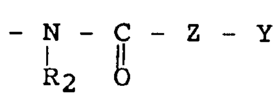

in which $R_2$ is $CH_3$, Z is $CH_2$ and Y is a 3,4-dimethoxy-phenyl, 3,4-dichlorophenyl, 4-trifluoromethylphenyl, 4-nitrophenyl or 4-benzothienyl radical and B represents the pyrrolidinyl radical;
the said compounds of formula (I) being able to be in all the possible enantiomeric and diastereoisomeric forms and in the form of addition salts with acids.

2. Compounds of formula (I) characterized in that in the group

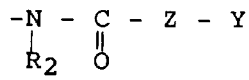

$R_2$ is a methyl or ethyl radical, Z is a $(CH_2)n_2$- group in which $n_2$ is 0 or 1,

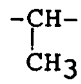

or $-CH_2-O-$ group, and in that in the group

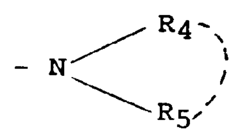

$R_4$ and $R_5$ form with the nitrogen atom to which they are linked the pyrrolidine heterocycle, as well as their addition salts with acids.

**3.** Compounds of formula (I) characterized in that in the group

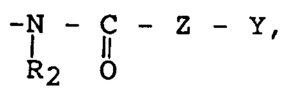

Y represents a phenyl radical optionally substituted by one or two identical radicals chosen from methyl, ethyl, methoxy and ethoxy radicals, chlorine or bromine atoms, trifluoromethyl and nitro radicals, or Y represents a naphthyl or benzo[b]thiophene radical, as well as their addition salts with acids.

**4.** Compounds of formula (I):

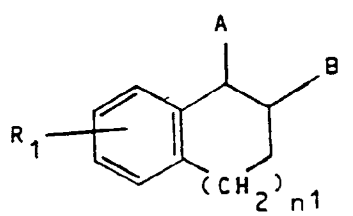

(I)

in which $R_1$ represents a hydrogen atom, a halogen atom, an alkyl or alkoxy radical containing 1 to 5 carbon atoms, nitro, amino, monoalkylamino or dialkylamino radicals, $n_1$ represents 1 or 2, and A and B are of trans configuration and are such that one of the substituents A or B represents a radical of formula:

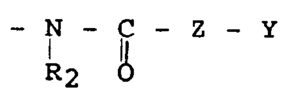

$R_2$ represents a hydrogen atom or an alkyl radical containing 1 to 5 carbon atoms, Z represents a linear alkylene chain $-(CH_2)n_2-$, $n_2$ being an integer which can vary between 0 and 5 or a branched alkylene chain containing 2 to 8 carbon atoms or a $-CH_2-O-$ group, Y represents a phenyl radical optionally substituted by one or more alkyl or alkoxy radicals containing 1 to 5 carbon atoms, halogen atoms, trifluoromethyl, nitro, amino, monoalkyl or dialkylamino radicals, a naphthyl radical, an indenyl radical, a thiazolyl, pyridinyl, oxazolyl, isoxazolyl, imidazolyl or thienyl radical or an indolyl, quinolyl, benzofurannyl, benzo[b]thienyl, benzimidazolyl, benoxazolyl or benzothiazolyl radical, all these radicals being optionally substituted by one or more identical or different radicals chosen from methyl or ethyl, methoxy or ethoxy radicals, a chlorine, bromine or fluorine atom, trifluoromethyl, nitro or phenyl radicals, and the other substituent A or

B represents a radical of formula:

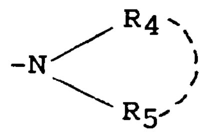

$R_4$ and $R_5$, identical or different, representing a hydrogen atom or an alkyl radical containing 1 to 5 carbon atoms or $R_4$ and $R_5$ form with the nitrogen atom to which they are linked a pyrrolidinyl, piperazinyl, piperidinyl, morpholinyl radical, and on condition that
either A represents a group of formula

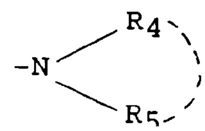

in which $R_4$ and $R_5$ have the previous meaning and B represents a group of formula

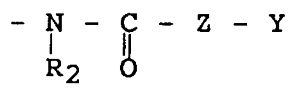

in which $R_2$, Z and Y have the previous meaning;
or Z represents $-(CH_2)n_2-$ in which $n_2$ is 0, 2, 3, 4 or 5, or a branched alkylene chain containing 2 to 8 carbon atoms or a $-CH_2-O-$ group;
or Y represents a phenyl radical substituted by one or more of the following radicals: alkyl containing 1 to 5 carbon atoms, alkoxy containing 2 to 5 carbon atoms, amino, monoalkyl or dialkylamino or Y represents a naphthyl radical, an indenyl radical, a thiazolyl, pyridinyl, oxazolyl, isoxazolyl, imidazolyl or thienyl radical, or an indolyl, quinolyl, benzofurannyl, benzo[b]thienyl, benzimidazolyl, benzoxazolyl or benzothiazolyl radical, all these radicals being optionally substituted by one or more identical or different radicals, chosen from methyl or ethyl, methoxy or ethoxy radicals, a chlorine, bromine or fluorine atom, trifluoromethyl, nitro or phenyl radicals, with the exception of the non-substituted benzothiophene;
or $R_1$ represents a nitro radical;
or $R_2$ represents an alkyl radical containing 4 or 5 carbon atoms;
the said compounds of formula (I) being able to be in all the possible enantiomeric and diastereoisomeric forms and in the form of addition salts with acids.

5. The compounds of formula (I), as defined in claim 1, in which:
either $R_1$ represents a hydrogen atom, $n_1$ represents 1, A represents the group

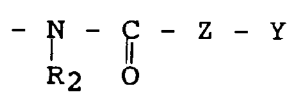

in which $R_2$ is $CH_3$, Z is $CH_2$ and Y is a 3,4-dimethoxy-phenyl, 4-nitrophenyl or 4-benzothienyl radical and B represents the pyrrolidinyl radical;
or $R_1$ represents a hydrogen atom, $n_1$ represents 2, A represents the group

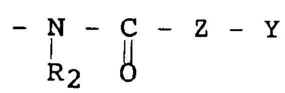

in which $R_2$ is $CH_3$, Z is $CH_2$ and Y is a 3,4-dimethoxy-phenyl, 3,4-dichlorophenyl, 4-trifluoromethylphenyl, 4-nitrophenyl or 4-benzothienyl radical and B represents the pyrrolidinyl radical; as well as their addition salts with acids.

6. - trans (±) 4-nitro N-methyl N-[2-(1-pyrrolidinyl) 1,2,3,4-tetrahydro 1-naphthyl] benzeneacetamide;
- trans (±) N-methyl N-[2-(1-pyrrolidinyl) 1,2,3,4-tetrahydro 1-naphthyl] 1-naphthaleneacetamide;
- trans (±) N-methyl N-[2-(1-pyrrolidinyl) 1,2,3,4-tetrahydro 1-naphthyl] 4-benzo[b]thiopheneacetamide;
as well as their addition salts with acids.

7. Preparation process for the products of formula (I) as defined in claim 1, characterized in that the product of formula (II):

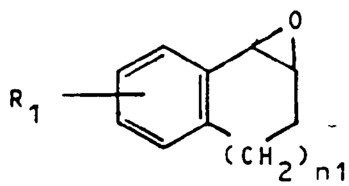

(II)

$R_1$ and $n_1$ having the meanings given previously, is condensed
- either with an amine of formula (III):

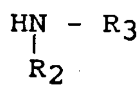

(III)

$R_2$ having the meaning given in claim 1 and $R_3$ being a protective group of the amine function, in order to obtain a product of formula (IV):

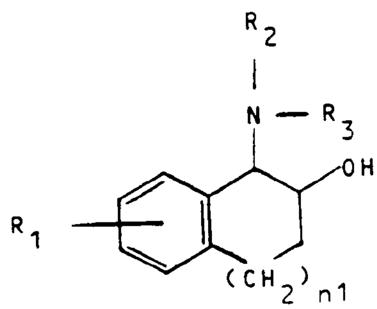

(IV)

the hydroxyl function of which is activated and which is condensed with an amine

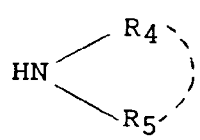

in which $R_4$ and $R_5$ have the meanings indicated in claim 1, in order to obtain a product of formula (V):

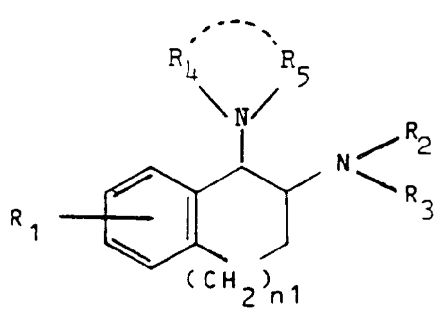

(V)

of which the $R_3$ protective group of the amine function is eliminated in order to obtain a product of formula (VI):

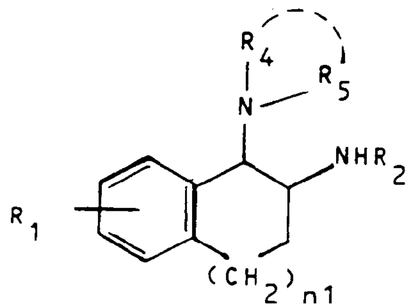

(VI)

which is treated with an acid of formula (VII) or a functional derivative of this acid:

Y-Z-COOH (VII)

in which Y and Z have the meanings indicated in claim 1, in order to obtain a product of formula (I) in which A represents

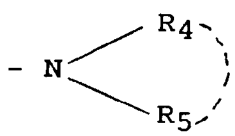 and B the group 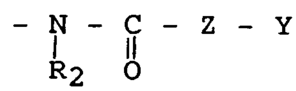

- or with an amine of formula

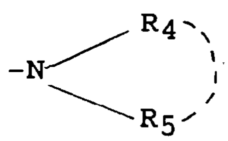

in order to obtain a product of formula (VIII):

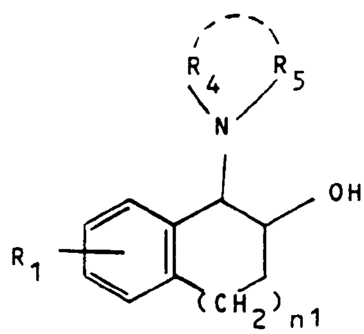

(VIII)

the hydroxyl function of which is activated and which is treated with an amine of formula (IX):

$NH_2R_2$ (IX)

in which $R_2$ has the previous meaning, in order to obtain a product of formula (X):

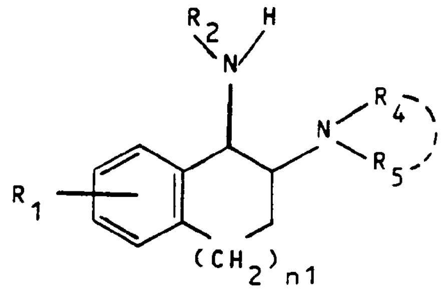

(X)

which is condensed with an acid of formula (VII) or a functional derivative of this acid:

Y-Z-COOH (VII)

in which Z and Y have the previous meanings, in order to obtain a product of formula (I) in which A represents the group

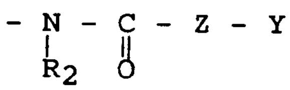 and B represents 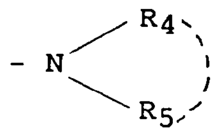

the said compounds of formula (I) being able to be resolved in order to obtain the optically active forms and treated, if desired, with a mineral or organic acid, in order to obtain the salts.

**8.** As medicaments, the products of formula (I), as defined in any one of claims 1 to 4, as well as their addition salts with pharmaceutically acceptable acids.

**9.** As medicaments, the products of formula (I) as defined in claim 5, as well as their addition salts with pharmaceutically acceptable acids.

**10.** As medicaments, the products of claim 6.

**11.** The pharmaceutical compositions containing as active ingredient at least one of the medicaments as defined by claims 8, 9 or 10.

**12.** As intermediate products, the products of formulae (V), (VI) and (X) as defined in claim 7 and such that for the products of formulae (V) and (VI), at least one of the following conditions is fulfilled:
- $R_4$ and $R_5$ do not represent a hydrogen atom;
- $R_1$ represents a nitro radical;
- $R_2$ represents an alkyl radical having 4 or 5 carbon atoms and for the products of formula (X), at least one of the following conditions is fulfilled:
  - $R_1$ represents a nitro radical;
  - $R_2$ represents an alkyl radical having 4 or 5 carbon atoms.